# NEW EUROPEAN PATENT SPECIFICATION

(11) **EP 3 692 101 B2**
(45) Date of publication and mention of the opposition decision: **04.11.2026**
(45) Mention of the grant of the patent: 12.01.2022
(21) Application number: 18780149.3
(22) Date of filing: 08.10.2018
(51) Int. Cl.: C09B 11/20, A61K 49/00, C07C 309/49, G01N 33/68, C07C 309/46

(54) **BRILLIANT BLUE (BBG) DYE DERIVATIVES AND STAINING COMPOSITIONS COMPRISING THE SAME FOR SELECTIVELY STAINING BIOLOGICAL SUBSTRATES**
BRILLANTBLAUE (BBG) FARBSTOFFDERIVATE UND FÄRBEZUSAMMENSETZUNGEN DAMIT ZUM SELEKTIVEN FÄRBEN VON BIOLOGISCHEN SUBSTRATEN
DÉRIVÉS DE COLORANT BLEU BRILLANT (BBG) ET COMPOSITIONS DE COLORATION LES COMPRENANT POUR COLORER SÉLECTIVEMENT DES SUBSTRATS BIOLOGIQUES

(30) Priority: 06.10.2017 WO PCT/EP2017/075562
(43) Date of publication of application: 12.08.2020
(73) Proprietor: Alfa Instruments S.r.l., 80026 Casoria (NA) (IT)
(72) Inventor: DAVID, Fabio, 80127 Napoli (IT)
(74) Representative: Porta & Consulenti Associati S.p.A.
(86) International application number: PCT/EP2018/077355
(87) International publication number: WO 2019/068935

(56) References cited:
- WO-A1-2006/062233
- WO-A1-2016/189475
- JP-A- 2006 265 438
- US-A- 5 132 439
- US-A- 5 273 906
- JU YUHONG ET AL: "Aqueous N- alkylation of amines using alkyl halides: direct generation of tertiary amines under microwave irradiation", GREEN CHEMISTRY, ROYAL SOCIETY OF CHEMISTRY, GB, vol. 6, no. 4, 1 January 2004 (2004-01-01), pages 219 - 221, XP009504977, ISSN: 1463-9262, DOI: 10.1039/B401620C

## Description

### Field of the invention

The present invention relates to dye molecules and, more specifically to brilliant blue (BBG) dye derivatives, and to staining compositions comprising said dye molecules that are particularly suitable for selectively staining biological substrates, such as structures in the posterior eye segment and proteins.

Said dye molecules may be used in ophthalmic surgery to help the surgeon to visualize structures in the posterior eye structures as well as in protein assays to determine one or more analytical parameter of a protein.

The present invention relates also to a new method for synthetizing the above dye molecules.

### Background of the invention

Biological substrates may be difficult to be discerned due to insufficient contrast.

For example, visualization and precise identification of structures in the posterior eye segment, such as the vitreous, epiretinal membrane (ERM), internal limiting membrane (ILM) and subretinal fluid, may be difficult since most of them are transparent/translucent.

Epiretinal membrane (ERM), also called macular pucker, is a disease of the eye in response to changes in the vitreous humor or more rarely, diabetes.

The internal limiting membrane (ILM), on the other hand, is the basal lamina of the inner retina that is formed by the footplates of the Müller cells and is the structural interface between the retina and the vitreous; the ILM is composed of collagen fibers, glycosaminoglycans, laminin, and fibronectin.

Surgical vitreoretinal procedures often require removal of these structures and therefore identification as well as precise delimitation of the same is of fundamental importance for ophthalmic surgeons.

For example, the ILM is a transparent, delicate structure composed, as mentioned above, by the footplates of the Müller cells. These cells are indispensable for the maintenance of the retina's normal function and structure. Partial or complete removal of the ILM may be however rendered necessary for treating different vitreoretinal conditions such as ERM, diabetic macular edemas, and macular holes.

Generally, removal of ILM is performed by means of a surgical technique of mechanical removal, called peeling , that can be extremely challenging even for experienced vitreoretinal surgeons in view of the difficulty to visualize the same as well as of the difficulty in identifying the right point of the posterior eye segment for starting the ILM peel, in visualizing the border between peeled and unpeeled ILM, and in establishing the extent of the ILM peel.

In protein assay tests, such as SDS-PAGE procedures, the contrast between proteins and a background surface is critical for a proper and clear visualization of proteins, for example when analyzing a protein extract, in order to determine one or more analytical parameters, such as protein quantity, molecular weight, identity, in a given sample.

### Related art

Staining is a technique widely known and used for biological substrates for the purpose of increasing color contrast through changing the color of some of the parts of a structure being observed, thus allowing for a clearer view of a given target object.

In recent years, many dyes molecules have been proposed to stain biological structures, thereby making them more visible.

For example, the application of dyes has been proposed to selectively stain retinal membranes in order to enable surgeons to perform peeling procedures with improved efficiency by making intraocular membranes more visible by means of an increase of the color contrast between different structures in the posterior eye segment.

In this context, dyes such as Indocyanine Green (hereinafter "ICG") and Trypan Blue (hereinafter "TB"), as well as other triphenylmethane dyes, such as Coomassie Brilliant Blue G-250 (hereinafter "BBG") and Coomassie Brilliant Blue R-250 (hereinafter "BBR"), have been for example proposed for staining retinal membranes in the surgical treatment of different vitreoretinal diseases.

EP 2 532 370, in the name of Fluoron GmbH, discloses a water-based, biocompatible, non-cytotoxic preparation for selectively coloring ILM and/or epiretinal membranes in the human or animal eye, and to a kit comprising said water-based preparation.

International patent application WO 2006/062233, in the name of National University Corporation Kyushu Corporation, discloses a staining composition for staining an ophthalmic membrane when performing the ophthalmic membrane removal, wherein the staining composition comprises a Brilliant Blue G (BBG) derivative as a primary component.

International patent application WO 2016/189475, in the name of AL.CHI.MI.A. S.r.I., discloses a dye molecule usable for making preparations for dyeing the internal limiting membrane of an eye, as well as at least several types of epiretinal membranes with the aim of creating a colour difference between the membranes dyed in that way. The dye molecule 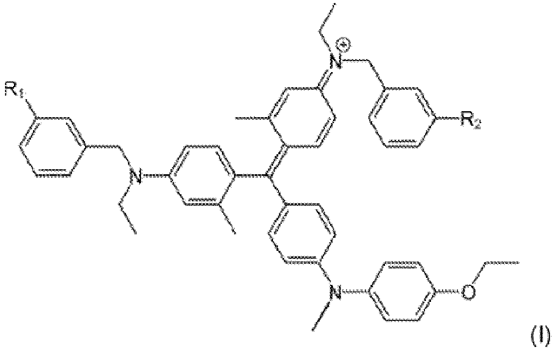 disclosed by this reference has the following structure (I):
where R₂ is constituted of an SO₃⁻ group (which forms an inner salt with the nearby positively charged nitrogen N atom) and R₁ is constituted of an SO₃⁻ group bound with an ionic bond to a hydrogen H atom or to another atom (for example, sodium Na) or to an ammonium NH₄ group or to a lysine salt or to an arginine salt or to a different monovalent cation.

US patents nos. 5,132,439 and 5,273,906, in the name of Promega Corporation, disclose 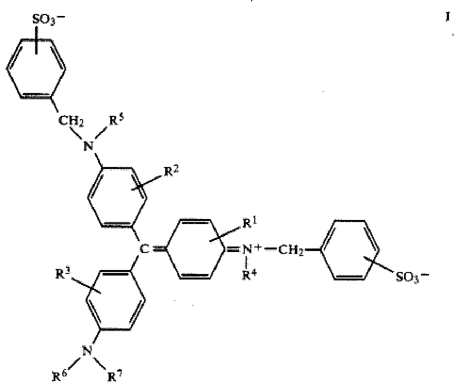 derivatives of Coomassie ^{®} dyes having the following structural formula (I): or a salt thereof, wherein R¹, R², and R³ are independently selected from the group consisting of hydrogen, alkyl and aryl groups; R⁴ and R⁵ are independently selected from the group consisting of alkyl and aryl groups; R⁶ is selected from the group consisting of hydrogen, substituted and unsubstituted alkyl, substituted and unsubstituted aryl, and substituted and unsubstituted arylalkyl groups; and R⁷ is a group which renders a Coomassie Blue dye more hydrophobic and is an acyl group of Formula II

-(C=O)R⁸ II

wherein R⁸ is selected from the group consisting of substituted and unsubstituted alkyl, substituted and unsubstituted aryl, and substituted and unsubstituted arylalkyl groups.

Ju at al. in "Aqueous N-alkylation of amines using alkyl halides: direct generation of tertiary amines under microwave irradiation", Green Chem., 2004, 6, 219-221, also disclose direct formation of tertiary amines via N-alkylation of amines by alkyl halides occurring in aqueous media under microwave irradiation.

According to this reference, a direct tert-amine forming protocol has reportedly been developed that is said to proceed rapidly via microwave-assisted N-alkylation of amines by alkyl halides achieving shorter reaction times and higher product yields in aqueous reaction medium.

As far as the alkylation of secondary amines is concerned, Table 2 of this reference indicates that alkylation of dibenzylamine with n-C₈H₁₇I may allow to obtain a n-C₈ alkyl derivative at a yield of 73% by operating at 1 mmol scale, MW power 250-300 Watt for 20-30 minutes.

In connection with the alkylation of secondary amines, Singh et al, "Modular Synthesis of Biaryl-Substituted Phosphine Ligands: Application in Microwave-Assisted Palladium-Catalyzed C-N Cross-Coupling Reactions", Eur. J. Org. Chem. 2015, 6515-6525, disclose that by combining biaryl-substituted monophosphine-based ligands with a palladium catalyst, C-N bond-forming reactions may be accomplished under microwave irradiation.

This reference also reports that even by using the above combination of monophosphine-based ligands and a palladium catalyst, the reaction of diphenylamine with either *o*- or *p*-bromoanisole does not afford the desired products probably as a result of strong steric hindrance caused by the two phenyl groups. Also, this reference reports that a blind reaction with various aprotic solvents in the absence of the above ligand gives no reaction as shown in Table 1, Entry 12.

### Summary of the invention

The Applicant has observed that the prior art solutions that propose of using dye molecules to stain biological substrates such as intraocular membranes, for example ERM and ILM, may present several limits.

In particular, the Applicant has noted that toxicological considerations may limit the possibility of using staining agents comprising high concentrations of dye molecules for staining intraocular membranes. The possibility of using limited amounts of these dyes, however, may lead to an insufficient contrast between the intraocular membranes of interest and the surrounding parts, thus rendering difficult to the surgeon the surgical procedure.

Additionally, the Applicant has noted that dye molecules such as TB and BBG are able to stain only certain intraocular membranes, especially when used at low concentrations. Therefore, in order to stain the desired intraocular membranes, a surgeon may be forced to select only certain dye molecules and use them only at certain high concentrations.

The Applicant has also experimentally observed in *ex-vivo* tests that substantial amounts of the BBG derivatives disclosed in the aforementioned International patent applications WO 2006/062233 and WO 2016/189475 permeate into the retina leading to an insufficient contrast between the intraocular membranes of interest and the retina and to possible toxic effects on the retinal tissue.

More particularly, it has been observed that as a consequence of an insufficient contrast between the intraocular membranes of interest and the retina, the surgical vitreoretinal procedures may cause a mechanical damage to the retina. In this regard, it has been in particular observed that inner limiting membrane (ILM) peeling may cause mechanical traumatic changes to the retinal nerve fiber layer and numerous cases of anatomical changes in the macula after ILM removal have been reported.

Finally, the Applicant has noted that the purity of dye molecules and the competitiveness and productivity of their synthesis processes are two opposing needs that may further limit the possibility of using the same. On one hand, in fact, the presence of chromogenic side products and impurities deriving from the dye molecules may impart to a staining composition an undesirable level of variation in terms of reproducibility, precision, and specificity of the staining due to diffusion phenomena to different sites than those expected. On the other hand, the need of expensive and long purification treatments due to the presence of said chromogenic side products and impurities may negatively affect the competitiveness and productivity of the synthesis process of the same dye molecules.

The Applicant has furthermore observed that the prior art solutions that propose to use dye molecules to stain proteins in protein assays may also present several limits.

In particular, the Applicant has noted that the degree of selectivity of the dye molecules to proteins may limit the precision and the detection limit of a protein assay. In particular, a reduced staining of the protein compared to the background may lead to the formation of a low color contrast, thus rendering the proteins to be analyzed hardly detectable and quantifiable, especially when they are present at low concentrations.

The Applicant has therefore addressed the problem of providing a new dye molecule and staining composition as well as a new process of synthesis capable of reducing and/or solving the above mentioned drawbacks.

In particular, the Applicant has, on one side, addressed the problem of providing new dye molecules and new staining compositions capable to specifically stain with high selectivity biological substrates, thus leading to an improved level of reproducibility, precision, and specificity in staining procedures. On the other side, the Applicant also addressed the problem of providing a new process for synthetizing dye molecules for biological substrates, capable of providing dye molecules at high level of purity and with short reaction times. The Applicant thereby achieves an improved balance between the need of providing a dye molecule at a high level of purity and of providing the same by means of a competitive process characterized by a high productivity.

According to the present invention, the Applicant has surprisingly and experimentally found that it is possible to achieve the desired specific staining with high selectivity of a biological substrate, in particular a highly selective staining of the internal limiting membrane (ILM), by using a new BBG derivative which shows a high affinity for these eye structures due to the presence of a highly lipophilic moiety.

The Applicant has also surprisingly and experimentally found a new method of synthesis of dye molecules capable of selectively staining biological substrates, which method allows both to eliminate or at least reduce the above discussed limits of the prior art solutions.

Therefore, the present invention relates, in a first aspect, to a dye molecule as defined in attached claim 1. More particularly, the present invention relates to a dye molecule having structure (I) 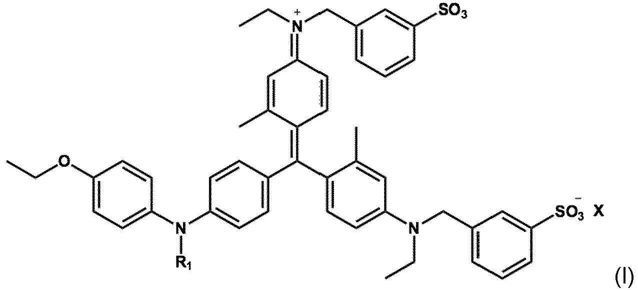 wherein
X represents an organic or inorganic cation; and
R₁ represents a substituted or unsubstituted benzyl group as defined in claim 1.

The Applicant has indeed unexpectedly found that with a dye molecule as defined above, it is possible to specifically stain biological substrates, such as structures in the posterior eye segment and proteins, with a high degree of selectivity that, compared to the dye molecules disclosed in the above prior art documents, lead to an improved level of reproducibility, precision, and specificity of staining procedures.

In a second aspect, the present invention relates also to a method of synthesis of a dye molecule as defined in attached claim 8. More particularly, the present invention relates to a method of synthesis of a dye molecule having the above structure (I),
the method comprising
a. forming a reaction mixture having a pH from 8 to 14 comprising a protic solvent and a molecule having structure (III) 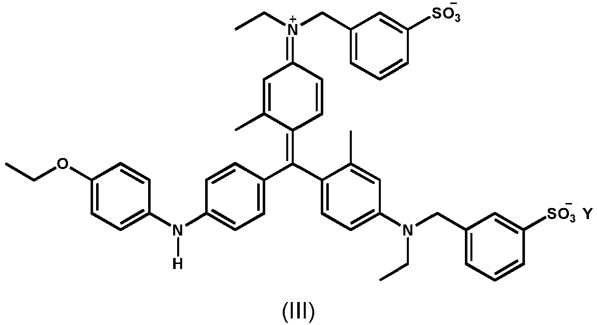 wherein Y represents an organic or inorganic cation;
   and
b. irradiating the reaction mixture resulting from step a. with microwave radiation comprising electromagnetic waves having power of from 5 W to 300 W in presence of an alkylating agent having structure (IV)

   R₁-Z (IV)

   wherein R₁ represents a substituted or unsubstituted benzyl group as defined in claim 1 and Z represents a leaving group; so as to obtain said dye molecule having structure (I), and
   wherein the alkylating agent is added to the reaction mixture in one or more aliquots in the course of step b.

Thanks to the new method of synthesis according to the present invention, the Applicant has indeed experimentally unexpectedly found that is possible to obtain dye molecules having structure (I), that is BBG derivatives, notwithstanding the fact that BBG may be considered as a derivative of diphenyl diamine, which has a basicity about 10 millionth lower than the dibenzylamine which has been alkylated by Ju at al. (the pKa of diphenylamine being 0.9 and the pKa of dibenzylamine being 8.52), and notwithstanding the fact that BBG has a steric hindrance which is far higher than the dibenzylamine disclosed by this reference.

The Applicant has also experimentally unexpectedly found that contrary to what was found by Singh et al, BBG alkylation may proceed in a protic solvent forming a C-N bond without any biaryl-substituted monophosphine-based ligands and without any palladium catalyst.

The above steps of the method of the present invention, in fact, are carried out without adding any biaryl-substituted monophosphine-based ligands and any palladium catalyst.

Overall, the method of synthesis according to the present invention allows to achieve the desired dye molecules with reaction times significantly shorter and with a level of purity higher than the methods of synthesis of similar compounds known in the prior art thereby achieving a high productivity and at the same time eliminating or at least markedly reducing the need of expensive purification treatments due to the presence of chromogenic side products and impurities.

Within the framework of the present description and in the subsequent claims, except where otherwise indicated, all the numerical entities expressing amounts, parameters, percentages, and so forth, are to be understood as being preceded in all instances by the term "about". Also, all ranges of numerical entities include all the possible combinations of the maximum and minimum values and include all the possible intermediate ranges, in addition to those specifically indicated herein below.

According to the invention, the substituted or unsubstituted benzyl group R₁ is selected from benzyl, 4-methylbenzyl, 2-methylbenzyl, 3-methylbenzyl, 3,5-dimethylbenzyl, 4-chlorobenzyl, 4-isopropylbenzyl, 2-(methyl)-1,3-dimethylbenzene, 4-(methyl)benzonitrile, 4-(trifluoromethyl)benzyl, 2,6-dichlorobenzyl, 2-chlorobenzyl, 1-(methyl)-2,3-dimethylbenzene, 3,4-dichlorobenzyl, 3,5-di-tert-butylbenzyl, 3-(methyl)benzonitrile, 4-fluorobenzyl, 1-(methyl)-3-(difluoromethyl)benzene, 3-(trifluoromethyl)benzyl, 4-bromobenzyl, 4-(methyl)benzoic acid, 2,3-dichlorobenzyl, 2,5-dichlorobenzyl, 4-iodobenzyl, 3-fluorobenzyl, 3,5-bis(trifluoromethyl)benzyl, 1-(methyl)-2-(difluoromethyl)benzene, 1-(methyl)-2,4-dimethylbenzene, 4-(methyl)-3-methylbenzonitrile, 4-methyl-benzamide, 4-(methylthio)benzyl, 2-(methyl)benzyl alcohol, 2-(trifluoromethyl)benzyl, 2-fluorobenzyl, 3-bromobenzyl, 2-bromobenzyl, 3,5-difluorobenzyl, 4-tert-butylbenzyl, 3-chlorobenzyl, 2-(methyl)benzonitrile, 1-methyl-4-(2,2,2-trifluoroethyl)benzene, 4-(methyl)-N,N-dimethylaniline hydrobromide, 2-iodobenzyl, 2,6-difluorobenzyl, 3-iodobenzyl, 3,5-dibromobenzyl, 3-(methyl)benzyl methyl ether, 3-(methyl)benzoic acid, 1-(methyl)-3-chloro-5-fluorobenzene, 3-fluoro-5-methylbenzyl, 2-(methyl)-1-methyl-4-(trifluoromethyl)benzene, 3-fluoro-4-methylbenzyl, 4-bromo-1-(methyl)-2-chlorobenzene, 4-fluoro-2-methylbenzyl, 4-phenoxybenzyl, 4-chloro-3-fluorobenzyl, 1-bromo-2-(methyl)-3-chlorobenzene, 2-fluoro-4-methylbenzyl, 4-fluoro-3-methylbenzyl, 2-[4-(methyl)phenyl]propionic acid, 2-chloro-6-fluorobenzyl, 4-(methyl)phenylboronic acid, 3-methoxybenzyl, 2,4,6-trifluorobenzyl, 2,4-difluorobenzyl, 3,4-difluorobenzyl, 4-(methyl)benzophenone, 2-methyl-3-(trifluoromethyl)benzyl, 2-chloro-4-fluorobenzyl, methyl 4-(methyl)benzoate, 4-chloro-2-fluorobenzyl, 2-fluoro-3-methylbenzyl, 3,5-dimethoxybenzyl, 1-bromo-4-(methyl)-2-chlorobenzene, 5-methyl-2-(trifluoromethyl)benzyl, 4-chloro-2,6-difluorobenzyl, 4-(methyl)-2-chloro-1-fluorobenzene, 5-fluoro-2-methylbenzyl, 3,4,5-trifluorobenzyl, 2,5-difluorobenzyl, 4-nitrobenzyl, 2-(methyl)-1-fluoro-3-methylbenzene, 2-fluoro-5-methylbenzyl, 3-chloro-5-(trifluoromethyl)benzyl, 2-chloro-5-fluorobenzyl, 4-(difluoromethoxy)benzyl, 2,3-difluorobenzyl, 3-chloro-2-fluorobenzyl, 2-[2-(methyl)phenyl]-2-methylpropanenitrile, 2-chloro-4-methoxybenzyl, ethyl 4-(methyl)benzoate, 2,3-difluoro-4-methylbenzyl, 2,6-difluoro-3-methylbenzyl, 4-chloro-3-(trifluoromethyl)benzyl, 5-chloro-2-fluorobenzyl, 4-(trifluoromethylthio)benzyl, 2-chloro-5-(trifluoromethyl)benzyl, 4-(methylsulfonyl)benzyl, methyl 3-(methyl)benzoate, 4-(trifluoromethoxy)benzyl, 3-bromo-5-fluorobenzyl, 4-chloro-2-(trifluoromethyl)benzyl, 3-phenylbenzyl, 2,3,5,6-tetrafluorobenzyl, 6-chloro-2-fluoro-3-methylbenzyl, 2-methyl-3-(trifluoromethyl)benzyl, 4-phenoxybenzyl, 2,3,5-trifluorobenzyl, 2,3,6-trifluorobenzyl, 3-nitrobenzyl, 3-(trifluoromethoxy)benzyl, [4-(methyl)phenoxy]acetonitrile, 2-bromo-1-(methyl)-4-tert-butylbenzene, 1-bromo-4-(methyl)-2-fluorobenzene, 2-bromo-4-fluorobenzyl, 2-chloro-6-(trifluoromethyl)benzyl, 1-(methyl)-2-phenoxybenzene, 4-cyano-2-fluorobenzyl, 3-chloro-2,4-difluorobenzyl, 4-(methyl)-2-fluorobenzonitrile, 5-bromo-2-(methyl)-1,3-difluorobenzene, 4-methyl-2,3,5,6-tetrafluorobenzyl, 2,4-dichloro-5-fluorobenzyl, 2-chloro-6-fluoro-3-methylbenzyl, 2-chloro-3,6-difluorobenzyl, 5-chloro-2-(trifluoromethyl)benzyl, 3-chloro-2,6-difluorobenzyl, 2,4,5-trifluorobenzyl, 2-phenylbenzyl, 3-fluoro-5-(trifluoromethyl)benzyl, 3-fluoro-4-(trifluoromethyl)benzyl, 3-(difluoromethoxy)benzyl, 4-bromo-2-fluorobenzyl, 2-bromo-4-(methyl)-1-fluorobenzene, 2,3,4-trifluorobenzyl, 2-bromo-1-(methyl)-3-fluorobenzene, 1-(methyl)-3-(methylsulfonyl)benzene, 4-bromo-3-(methyl)benzonitrile, 3-methyl-4-nitrobenzyl, 3-cyano-4-fluorobenzyl, tert-butyl 3-(methyl)benzoate, 2-bromo-5-fluorobenzyl, 2-fluoro-4-(trifluoromethyl)benzyl, 2-fluoro-3-(trifluoromethyl)benzyl, 2,3,4,5,6-pentafluorobenzyl, 4-bromo-1-(methyl)-2-ethylbenzene, 2-bromo-1-(methyl)-3-(trifluoromethyl)benzene, 2-(methyl)-4-chloro-1-methoxybenzene, 3-bromo-2-fluorobenzyl, 1-(methyl)-2-methyl-4-(trifluoromethoxy)benzene, 4'-methyl-2-biphenylcarbonitrile, 4-fluoro-3-(trifluoromethyl)benzyl, 2-fluoro-5-(trifluoromethyl)benzyl, 2-nitrobenzyl, 4-(methyl)-2-methoxybenzonitrile, 2-(methyl)-5-fluorobenzonitrile, 1-(methyl)-3-methyl-5-(trifluoromethoxy)benzene, 5-fluoro-2-(trifluoromethyl)benzyl, 1-(methyl)-2-(difluoromethoxy)benzene, 4-fluoro-2-(trifluoromethyl)benzyl, 4-(methyl)-3-methoxybenzonitrile, 4-methoxy-3-(trifluoromethyl)benzyl, 3-fluoro-4-methoxybenzyl, 2-(trifluoromethoxy)benzyl, 3-(methyl)-4-methoxybenzonitrile, 3-bromo-2-(methyl)benzonitrile, 4-bromo-1-(methyl)-2-(trifluoromethyl)benzene, methyl 3-(methyl)-4-chlorobenzoate, 2-(methyl)-3-fluorobenzonitrile, 1-(methyl)-3-(4-fluorophenoxy)benzene, 2-(methyl)-1-fluoro-4-methoxybenzene, 2-bromo-6-(trifluoromethyl)benzyl, 4-fluoro-3-methoxybenzyl, 5-fluoro-2-methoxybenzyl, 3-fluoro-5-methoxybenzyl, 2-bromo-5-methoxybenzyl, 2,3,5,6-tetrafluoro-4-(trifluoromethyl)benzyl, phenacyl 4-(methyl)phenylacetate, 2-fluoro-6-(trifluoromethyl)benzyl, 2-hydroxy-5-nitrobenzyl, 2-fluoro-3-methoxybenzyl, 4-methyl-N,N-dimethyl-benzenesulfonamide, 4-bromo-2-(methyl)-1-methoxybenzene, tert-butyl [2-(methyl)phenoxy]acetate, 4-(methyl)-1-ethoxy-2-(trifluoromethyl)benzene, 4-chloro-3-(trifluoromethoxy)benzyl, 2-fluoro-6-methoxybenzyl, 3-fluoro-4-nitrobenzyl, 2-bromo-1-methyl-5-methoxy-3-methylbenzene, methyl 4-(methyl)-3-methoxybenzoate, 1-bromo-2-(methyl)-4,5-dimethoxybenzene, (4-(methyl)phenyl)(4-(prop-2-yn-1-yloxy)phenyl)methanone, (4-(methyl)phenyl)(4-(prop-2-yn-1-yloxy)phenyl)methanone, tert-butyl 2,4-difluoro-3-methoxybenzyl, 2,6-difluoro-3-methoxybenzyl, 2-nitro-4-(trifluoromethyl)benzyl, 4-fluoro-3-nitrobenzyl, 4-methyl-3-nitrobenzoic acid, methyl 3-bromo-5-(methyl)benzoate, 2-fluoro-5-(trifluoromethoxy)benzyl, methyl 2-bromo-4-(methyl)benzoate, methyl 3-(methyl)-2-methoxybenzoate, 4-bromo-1-(methyl)-2-(methylsulfonyl)benzene, methyl 4-bromo-2-(methyl)benzoate, 4-fluoro-2-nitrobenzyl, 4-bromo-1-(methyl)-2-nitrobenzene, 2-[(phenylsulfonyl)methyl]benzyl, ethyl 4-bromo-2-(methyl)benzoate, methyl 2-(methyl)-4-nitrobenzoate, 1-(methyl)-2-fluoro-3-(trifluoromethoxy)benzene, methyl 3-bromo-2-(methyl)benzoate, 3-ethoxy-2,4-difluorobenzyl, 4,5-dimethoxy-2-nitrobenzyl, 4-[2-(methyl)phenoxy]tetrahydropyran, 1-[3-(methyl)phenyl]-1H-pyrrole, 2-[4-(methyl)phenyl]-5-methyl, 4-benzyl-acetic acid.

The present invention may present in one or more of the above aspects one or more of the characteristics disclosed hereinafter.

Preferably, the substituted or unsubstituted benzyl group is selected from benzyl, 4-(trifluoromethyl)benzyl, 3,5-bis(trifluoromethyl)benzyl, 4-benzyl-acetic acid, 3,4-dichlorobenzyl or 4-(tert-butyl)benzyl, more preferably is a benzyl group.

Preferably, in the dye molecule having structure (I), X represents an organic or inorganic cation selected from the group consisting of H⁺, NH₄⁺, an ion selected from the group consisting of Na⁺, Mg⁺⁺, K⁺, Ca⁺⁺, a lysinium cation or an argininium cation. More preferably, X represents Na⁺ or K⁺.

In particular, the Applicant has found that dye molecules having the R₁ and X groups as above defined show particularly high selectivity for biological substrates, such as intraocular membranes and proteins.

In a preferred embodiment, the present invention relates also to a staining composition comprising said dye molecule having structure (I), a pharmaceutically acceptable salt, or a hydrate thereof.

Thanks to its capability of specifically stain biological substrates with high selectivity, the dye molecule having structure (I) may indeed be advantageously formulated in a staining composition that result suitable for staining techniques of biological substrates.

Preferably, the staining composition according to the present invention comprises from 0.0001% to 0.5% by weight, more preferably from 0.010% to 0.1% by weight, of the dye molecule having structure (I), a pharmaceutically acceptable salt, or a hydrate thereof.

Preferably, the staining composition according to the present invention further comprises at least one density adjuster.

Preferably, said density adjuster is selected from the group consisting of D₂O, monosaccharides, disaccharides, polysaccharides, xanthophylles and polymers.

In the context of the staining composition according to the present invention, said density adjuster preferably comprises at least one xanthophyll. More preferably said xanthophyll is lutein.

Lutein is a natural polymeric xanthophyll that is advantageously used in the staining composition according to the present invention in view of its capacity to absorb and attenuate the damaging blue light before it reaches the retinal cells.

Preferably, the staining composition according to the present invention comprises from 0.2% to 4.0% by weight, more preferably from 0.5% to 3.0% by weight, of at least one density adjuster.

The staining composition according to the invention preferably comprises at least one second dye molecule different from the dye molecule having structure (I), a pharmaceutically acceptable salt, or a hydrate thereof.

Preferably, said second dye molecule may be selected from the group consisting of: Coomassie Brilliant Blue G-250, Coomassie Brilliant Blue R-250, indocyanine green, infracyanine green, trypan blue, patent blue, bromophenol blue, fast green, indigo carmine, evans blue, light green, triamcinolone, crystal violet, fluorescein, fluorometholone acetate and congo red and combinations thereof. More preferably, said second dye molecule is trypan blue.

Preferably, the staining composition according to the present invention comprises from 0.001% to 0.5% by weight, more preferably from 0.05% to 0.25% by weight, of at least one second dye molecule.

Preferably, the staining composition according to the present invention comprises one or more of the other possible ingredients known in the pharmaceutical art for staining compositions.

Preferably, the staining composition according to the present invention comprises at least one component selected from the group consisting of buffering agents, osmolarity adjusters, preservatives, antimicrobials and/or sequestering agents, and mixtures thereof.

Examples of buffering agents which can be used in the present invention are selected from the group consisting of sodium acetate, sodium phosphate, sodium hydroxide, sodium citrate, sodium carbonate, sodium borate, sodium bicarbonate, potassium phosphate, potassium citrate, potassium carbonate, phosphoric acid, boric acid, hydrochloric acid, acetic acid and magnesium chloride, and mixtures thereof. Preferably, said buffering agents comprise a phosphate buffering agent.

The buffering agents help to maintain the pH of the staining composition as close as possible to the physiological pH. This action is advantageous for achieving a good tolerability of the staining composition and for preserving the effectiveness thereof.

Preferably, polymers that may be used in the staining composition according to the invention are selected from the group consisting of polyethers, polyvinyl alcohol, polyesters, polyacrylic acid copolymers, polyvinyl pyrrolidone, cellulose derivatives polyethylene glycols, polysorbate, xanthan gum and glycosaminoglycans.

Preferably said cellulose derivatives are selected from the group consisting of methylcellulose and carboxymethylcellulose.

Preferably, said glycosaminoglycans are selected from the group consisting of sodium hyaluronate and chondroitin sulphate.

In a preferred embodiment, the staining composition according to the present invention comprises from 0.01% to 0.1% by weight of the dye molecule having structure (I), a pharmaceutically acceptable salt, or a hydrate thereof, from 0.5% to 3.0% by weight lutein, from 0% to 0.25% by weight of trypan blue.

Preferably, the staining composition according to the present invention has a physiologically acceptable osmolarity, preferably comprised between 250 and 350 mOsm/kg. Within the context of the present invention, osmolarity of the staining composition according to the present invention may be measured by cryoscopic osmometry, using for example an Osmomat 030-D automatic cryoscopic osmometer (Gonotech, GmbH, Berlin, Germany).

Preferably, the staining composition according to the present invention has a density comprised between 1.012 and 1.015 g/ml. Within the context of the present invention, the density of the staining composition may be measured by capillary pycnometry for example using a single capillary pycnometer having a bulb capacity of 10 mL, calibrated using bidistilled water.

The staining composition comprising the dye molecule having structure (I) according to the present invention finds applications in any field where selective staining of biological substrates may be useful, including, but not limited to, methods of ophthalmic surgery that comprise the staining an ocular membrane or structure as well as to methods of staining proteins in protein assays aimed at determining one or more analytical parameter of said proteins.

In a preferred embodiment, the present invention therefore relates also to a staining composition as described above, for use in a method of ophthalmic surgery comprising staining an ocular membrane or structure.

By selectively staining such biological substrates, indeed, the staining composition according to the invention renders the same more visible and may therefore help the surgeon to better visualize the same in the posterior eye segment.

In the context of the present invention, disclosed is therefore also a method of ophthalmic surgery comprising staining an ocular membrane or structure with the staining composition according to the present invention.

Preferably, said ocular membrane or structure comprises the anterior and posterior capsule, corneal epithelium and endothelium, epiretinal membrane, internal limiting membrane, vitreous or posterior hyaloid.

Preferably, said method of ophthalmic surgery comprises the removal of an ocular membrane or structure.

Preferably, said staining composition for use in a method of ophthalmic surgery is in the form of aqueous suspension or solution in a pharmaceutically acceptable ophthalmic carrier.

As disclosed above, the staining composition according to the present invention finds application also in the field of the methods for staining proteins in protein assays aimed at determining one or more analytical parameter of said proteins.

In a preferred embodiment, the present invention therefore relates also to a method of staining proteins in a sample comprising the step of contacting said sample with the staining composition according to the present invention.

The high selectivity of the dye molecule according to the invention, indeed, provides a high color contrast of the proteins compared to a background, thus rendering the analyzed proteins clearly detectable and quantifiable, even when they are present at low concentrations.

Preferably, said staining is performed to determine one or more analytical parameter of said proteins. Preferably, said analytical parameter is selected from the group consisting of molecular weight, quantity, identity.

As disclosed above, in a second aspect the present invention relates also to a method of synthesis of a dye molecule having structure (I).

Said method comprises the step a. of forming a reaction mixture having a pH from 8 to 14 comprising a solvent and a molecule having structure (III).

Preferably, the above protic solvent may be selected from methanol, water, ethanol, n-propanol, t-butanol, ammonia, and mixtures thereof.

More preferably, said solvent comprises a mixture of methanol and water, wherein water represents from 1% to 99% by volume of said mixture.

In a preferred embodiment and as far as the solvent is concerned, the reaction mixture exclusively comprises a protic solvent, in the sense that the only solvent used is a protic one.

In another preferred embodiment, the reaction mixture may also comprise an aprotic solvent.

Preferably, said aprotic solvent may be selected from dimethylformamide, acetonitrile, acetone, dimethylsulfoxide, ethyl acetate and mixtures thereof.

Preferably, the reaction mixture of step a. shows a pH of from 10 to 14.

Preferably, said reaction mixture may comprise at least one organic or inorganic base selected from alkaline and alkaline earth hydroxides, sodium or potassium carbonate, sodium or potassium bicarbonate, triethylamine, 4-(dimethylamino)pyridine, pyridine, N,N-diisopropylethylamine and mixtures thereof.

Preferably, in the molecule having structure (III), Y represents an organic or inorganic cation selected from the group consisting of H⁺, NH₄⁺, an ion selected from the group consisting of Na⁺, Mg⁺⁺, K⁺, Ca⁺⁺, a lysinium cation or an argininium cation. More preferably, Y represents Na⁺ or K⁺,

Said method for the synthesis of a dye molecule having structure (I) comprises the step b. of irradiating the reaction mixture resulting from step a. with microwave radiation comprising electromagnetic waves having power of from 5 W to 300 W in presence of an alkylating agent having structure (IV).

Preferably, said microwave radiation has power of from 50 W to 200 W, more preferably of from 100 W to 180 W.

Preferably, the electromagnetic waves have frequency of from 300 MHz to 300 GHz.

Preferably, said step b. is performed at a temperature of from 30°C to 100°C, more preferably of from 60°C to 90°C.

The alkylating agent having structure (IV) of step b. carries a leaving group Z which is preferably selected from the group consisting of halides.

More preferably said halides are selected from chlorides, bromides, iodides.

Preferably, in the synthesis method according to the present invention the molar ratio between the alkylating agent having the structure (IV) and the molecule having the structure (III) ranges from 100:1 to 1:1. More preferably, said molar ratio ranges from 50:1 to 1:1

In a preferred embodiment, the alkylating agent is added to the reaction mixture in 1 to 10 aliquots, more preferably in 1-7 aliquots, in the course of step b.

In this way, it is possible to advantageously further improve the method in terms of reduction of the reaction time and formation of side products.

Preferably, said method for the synthesis of a dye molecule having structure (I) comprises the step of purifying said dye molecule having structure (I) resulting from step b.

Preferably, said step of purifying said dye molecule having structure (I) comprises at least one operation selected from the group consisting of filtering, washing, centrifuging, drying, and lyophilizing.

### Brief description of the figures

Further characteristics and advantages of the invention will become clearer from the following description of some preferred embodiments thereof, made hereinafter, for indicating and not limiting purposes, with reference to the attached drawings. In such drawings:
- figure 1 shows the 1H-NMR spectrum of the ethyl-BBG according to Example 2;
- figure 2 shows the RP-HPLC chromatogram of the ethyl-BBG according to Example 2;
- figure 3 shows the 1H-NMR spectrum of the propyl-BBG according to Example 2;
- figure 4 shows the 1H-NMR spectrum of the 2-hydroxy-ethyl-BBG according to Example 2;
- figure 5 shows the 1H-NMR spectrum of the 2-methoxy-ethyl-BBG according to Example 2;
- figure 6 shows the 1H-NMR spectrum of the benzyl-BBG according to Example 4;
- figure 7 shows the RP-HPLC chromatogram of the benzyl-BBG according to Example 4;
- figure 8 shows the 1H-NMR spectrum of the methyl-BBG according to Example 5;
- figure 9 shows the RP-HPLC chromatogram of the methyl-BBG according to Example 5;
- figure 10 shows the SDS-PAGE and spot intensity calculation analysis of E.coli beta-galactosidase - 116.3 kDa, bovine serum albumin - 66.5 kDa, chicken ovalbumin - 42.7 kDa and equine cytochrome C - 12,384 Da, using the Benzyl-BBG according to Example 4, the Ethyl-BBG according to Example 2, compared with BBG as reference staining agent;
- figure 11 shows a diagram illustrating the ex-vivo dye distribution in the ILM and the retina of swine eyes of a dye according to the invention and of two comparative dyes according to the prior art.

### Detailed description of the currently preferred embodiments

### EXAMPLE 1 (comparative) - General synthesis procedure of dye molecules having structure (I) wherein R₁ is ethyl, 2-hydroxyethyl-, 2-methoxy-ethyl- or propyl-without microwaves radiation

In a glass reactor of 250 ml equipped with a dropping funnel and a magnetic stirrer, 3.35 mmol (2.86 g) of BBG (Sigma-Aldrich 90% purity), 133.98 mmol of the appropriate alkyl-bromide alkylating agent (Ethyl-bromide, 2-Bromoethanol, 2-Bromoethyl methyl ether or Propyl-bromide), 133.98 mmol of NaOH (5.36 g) and 0.6 mmol (100 mg) of KI were added to 80 ml of 1/1 v/v methanol/water mixture. The mixture thus obtained was stirred at room temperature, while adding a further amount of 535.92 mmol of the appropriate alkyl-bromide (40 ml), 535.92 mmol of NaOH (21.44 g) and additional 40 ml of 1/1 v/v methanol/water mixture.

Said additional alkyl-bromide, NaOH and additional methanol/water mixture were added to the reaction mixture in several aliquots during the reaction at time intervals of 24 hours starting from the beginning of the reaction as shown in Table 1.

A sample of the reaction mixture was also withdrawn from the reactor every 24 hours and analyzed by HPLC (by using an Agilent 1260 Infinity II HPLC instrument, equipped with a Diode Array Detector Agilent G7115A as detector and using a ternary eluent consisting of methanol, acetonitrile and triethylamine 0.3% at pH 3.0 with HCOOH and a column Eurosphere II 100-3 C18 from Knauer) for detecting the formation of the target compounds and the reaction was continued for 144-168 hours until complete conversion of the BBG of the starting mixture.

**Table 1**

| **Time (h)** | **Alkyl-bromide (mmol)** | **NaOH (mmol)** | **methanol/water mixture 1/1 v/v (ml)** |
|---|---|---|---|
| 24 | 133.98 | 133.98 | 10 |
| 48 | 133.98 | 133.98 | 10 |
| 72 | 133.98 | 133.98 | 10 |
| 96 | 133.98 | 133.98 | 10 |
| 168 | - | - | - |

### Synthesis of Ethyl-BBG

Ethyl-BBG was synthetized by following the general synthesis procedure reported above, using ethyl bromide as alkylating bromide (alkylating agent).

The Ethyl-BBG was obtained in a reaction yield of 90% (determined by HPLC).

### Synthesis of Propyl-BBG

Propyl-BBG was synthetized by following the general synthesis procedure reported above, using propyl-bromide as alkylating bromide (alkylating agent).

The propyl-BBG was obtained in a reaction yield of 90% (determined by HPLC).

### Synthesis of 2-Hydroxy-ethyl-BBG

2-hydroxy-ethyl-BBG was synthetized by following the general synthesis procedure reported above, using 2-Bromoethanol as alkylating bromide (alkylating agent).

The 2-hydroxy-ethyl-BBG was obtained in a reaction yield of 85% (determined by HPLC).

### Synthesis of 2-Methoxy-ethyl-BBG

2-methoxy-ethyl-BBG was synthetized by following the general synthesis procedure reported above, using 2-Bromoethyl methyl ether as alkylating bromide (alkylating agent).

The 2-methoxy-ethyl-BBG was obtained in reaction yield of 87% (determined by HPLC).

### EXAMPLE 2 (not according to the invention) - General synthesis procedure of a dye molecule having structure (I) wherein R₁ is ethyl-, 2-hydroxyethyl-, 2-methoxy-ethyl-and propyl- with a method including a microwaves radiation

Microwave-assisted reactions were carried out in a CEM Discover System. 0.04 mmol (33 mg) of BBG (Sigma-Aldrich 90% purity), 0.8 mmol of the appropriate alkyl-bromide alkylating agent, 0.2 mmol of NaOH (8 mg) and 0.06 mmol (10 mg) of KI were added to 4 ml of 1/1 v/v methanol/water mixture. The mixture thus obtained was irradiated at 80°C with 150 W of a microwave radiation at 2455 MHz while adding a further amount of alkyl-bromide (13.2 mmol) and 8.8 mmol of NaOH (352 mg).

Said additional alkyl-bromide and NaOH were added to the reaction mixture in several aliquots during the reaction at time intervals of 1-5 minutes starting from the beginning of the irradiation as shown in Table 2.

A sample of the reaction mixture was also withdrawn from the reactor every 1-5 minutes and analyzed by HPLC (by using an Agilent 1260 Infinity II HPLC instrument, equipped with a Diode Array Detector Agilent G7115A as detector and using a ternary eluent consisting of methanol, acetonitrile and triethylamine 0.3% at pH 3.0 with HCOOH and a column Eurosphere II 100-3 C18 from Knauer) for detecting the formation of the target compounds. The reaction was continued for 0.5-1 hours starting from the beginning of the irradiation, until complete conversion of the BBG of the starting mixture.

**Table 2**

| Time from the beginning of the irradiation (min) | **alkyl-bromide (mmol)** | NaOH (mmol) |
|---|---|---|
| 30 | 1.2 | 0.4 |
| 60 | 1.6 | 0.8 |
| 90 | 1.6 | 1.2 |
| 120 | 1.6 | 1.6 |
| 150 | 2 | 1.6 |
| 180 | 2.4 | 1.6 |
| 210 | 2.8 | 1.6 |
| 240 | - | - |

### Synthesis of Ethyl-BBG

Ethyl-BBG was synthetized by following the general synthesis procedure reported above, using ethyl bromide as alkylating bromide (alkylating agent).

The Ethyl-BBG was obtained in a reaction yield of 98% (determined by HPLC) and, after purification by flash-chromatography on silica gel using as eluent gradient of CH₂Cl₂/MeOH, it was analyzed by means of 1H-NMR on a Varian Inova 200 MHz spectrometer using DMSO-d6 as solvent.

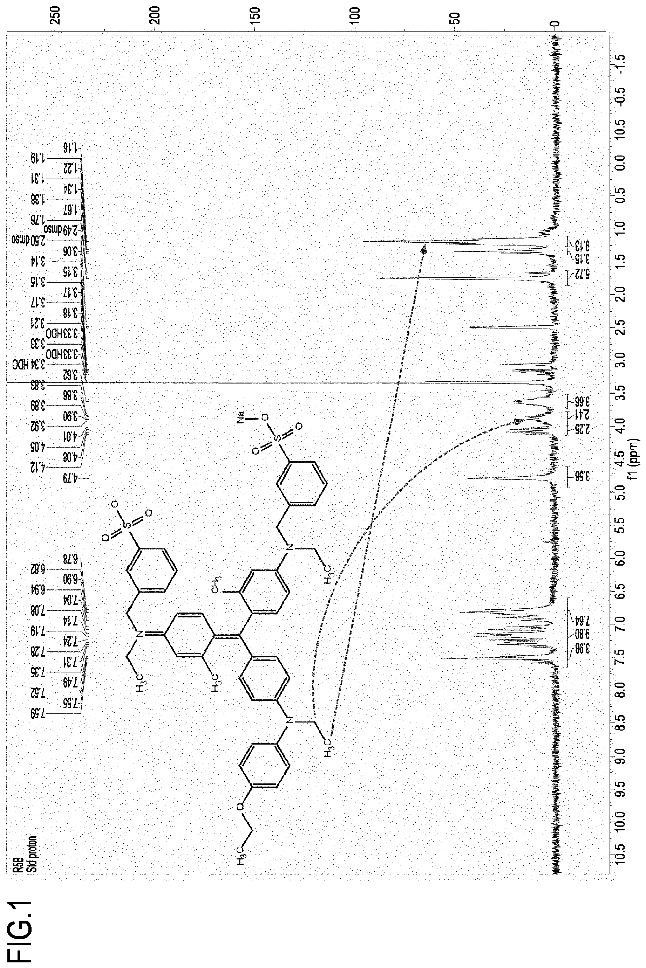

Figure 1 shows the 1H-NMR spectrum of the product obtained, in which the following peak attributions were made: ¹H-NMR (DMSO-d6) d ppm: 7.65-7.42 (m, 4H, Ar***H***); 7.39-6.98 (m, 10H, Ar***H***); 6.96-6.68 (m, 8H, Ar***H***); 4.78 (br s, 2H, Ar-***CH₂***-N); 4.13-3.99 (m, 2H, CH₃-***CH₂***-O); 3.96-3.79 (m, 2H, CH₃-***CH₂***-N-Ar₂); 3.74-3.52 (m, 4H, CH₃-***CH₂***-N-Ar); 1.75 (br s, 6H, ***CH₃***-Ar); 1.40-1.28 (m, 3H, ***CH₃***-CH₂-O); 1.27-1.08 (m, 9H, ***CH₃***-CH₂-N).

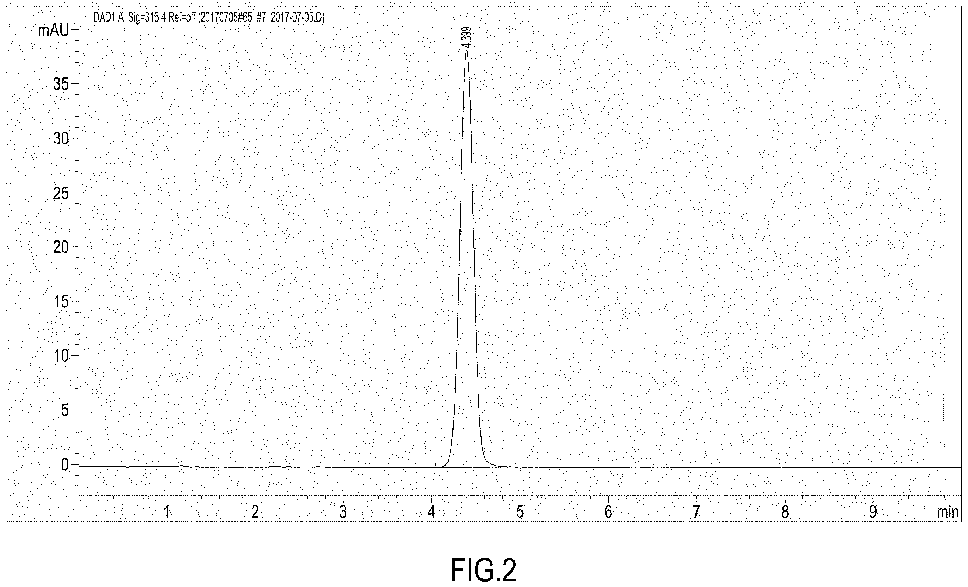

The Ethyl-BBG thus purified was also analyzed by means of RP-HPLC (by using an Agilent 1260 Infinity II HPLC instrument, equipped with a Diode Array Detector Agilent G7115A as detector and using a ternary eluent consisting of methanol, acetonitrile and triethylamine 0.3% at pH 3.0 with HCOOH and a column Eurosphere II 100-3 C18 from Knauer). Figure 2 shows the RP-HPLC chromatogram of the product obtained, showing at an elution time of 4.399 min. of the peak of the Ethyl-BBG.

Finally, the Ethyl-BBG thus purified was also analyzed by ESI-MS (API 2000 LC/MS/MS System from Applied Biosystems). From the analysis of the fragmentation of the Ethyl-BBG is evident a molecular peak at 860.3 (M-23(Na⁺)+1(H⁺).

### Synthesis of Propyl-BBG

Propyl-BBG was synthetized by following the general synthesis procedure reported above, using propyl-bromide as alkylating bromide (alkylating agent).

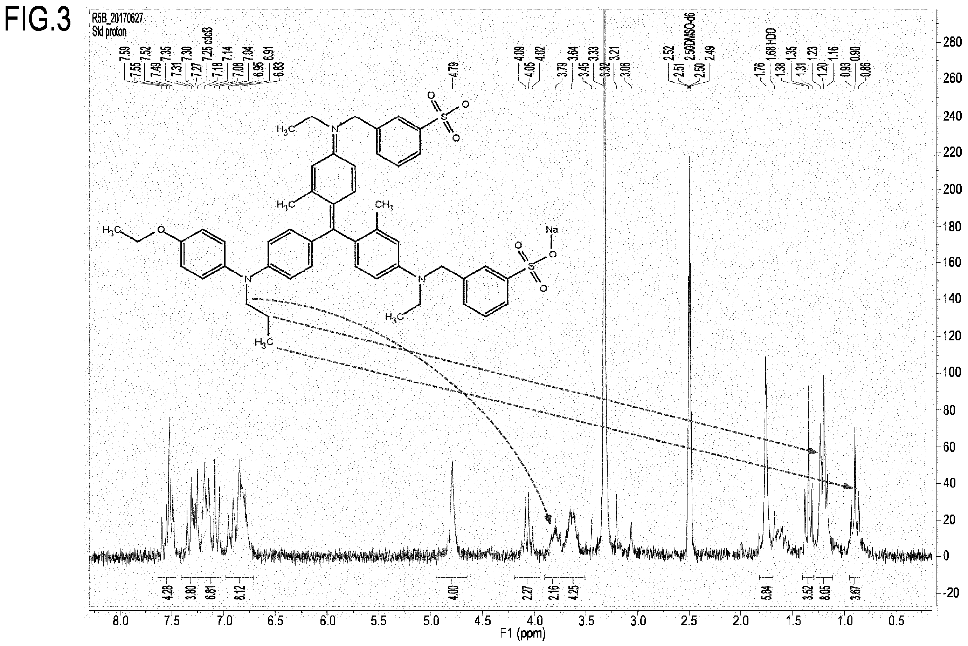

The propyl-BBG was obtained in a reaction yield of 90% (determined by HPLC) and, after purification by flash-chromatography on silica gel using as eluent gradient of CH₂Cl₂/MeOH, it was analyzed by means of 1H-NMR on a Varian Inova 200 MHz spectrometer using CD3OD or DMSO-d6 as solvent. Figure 3 shows the 1H-NMR spectrum of the product obtained, in which the following peak attributions were made: ¹H-NMR (DMSO-d6) d ppm: 7.63-7.45 (m, 4H, Ar***H***); 7.37-7.01 (m, 10H, Ar***H***); 6.98-6.74 (m, 8H, Ar***H***); 4.79 (br s, 2H, Ar-***CH₂***-N); 4.15-3.99 (m, 2H, CH₃-***CH₂***-O); 3.88-3.72 (m, 2H, CH₃-CH₂-***CH₂***-N-Ar₂); 3.72-3.55 (m, 4H, CH₃-***CH₂***-N-Ar); 1.76 (br s, 6H, ***CH₃***-Ar); 1.43-1.29 (m, 3H, ***CH₃***-CH₂-O); 1.28-1.11 (m, 8H, 2 x ***CH₃***-CH₂-N and CH₃-***CH₂***-CH₂-N-Ar₂); 0.98-0.82 (***CH₃***-CH₂-CH₂-N-Ar₂).

### Synthesis of 2-Hydroxy-ethyl-BBG

2-hydroxy-ethyl-BBG was synthetized by following the general synthesis procedure reported above, using 2-Bromoethanol as alkylating bromide (alkylating agent).

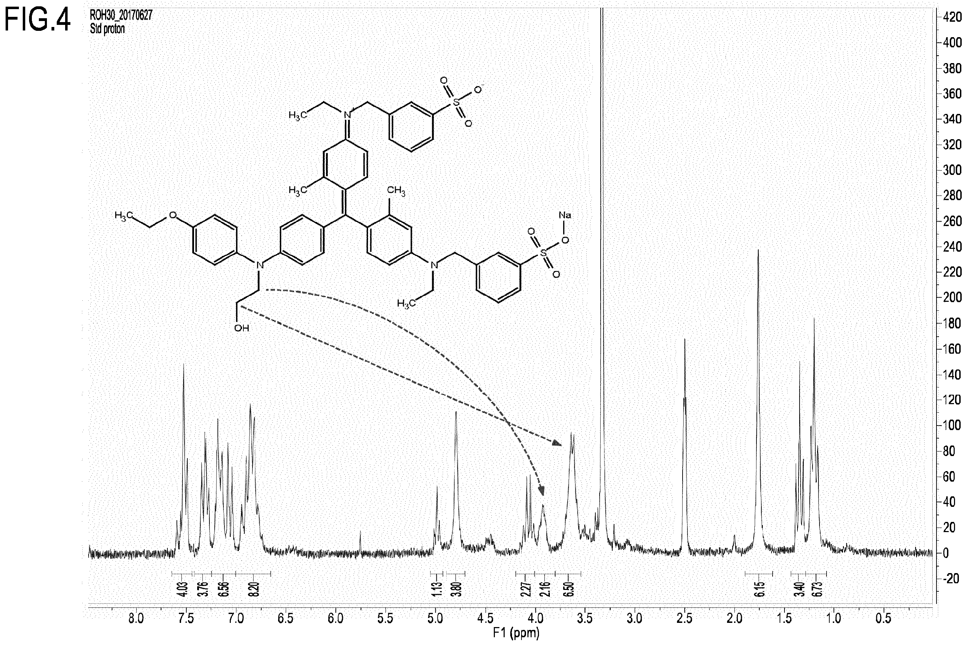

The 2-hydroxy-ethyl-BBG was obtained in a reaction yield of 88% (determined by HPLC) and, after purification by flash-chromatography on silica gel using as eluent gradient of CH₂Cl₂/MeOH, it was analyzed by means of 1H-NMR on a Varian Inova 200 MHz spectrometer using CD3OD or DMSO-d6 as solvent. Figure 4 shows the 1H-NMR spectrum of the product obtained, in which the following peak attributions were made: ¹H-NMR (DMSO-d6) d ppm: 7.63-7.46 (m, 4H, Ar***H***); 7.40-7.00 (m, 10H, Ar***H***); 6.98-6.72 (m, 8H, Ar***H***); 4.80 (br s, 2H, Ar-***CH₂***-N); 4.17-4.00 (m, 2H, CH₃-***CH₂***-O); 3.98-3.85 (m, 2H, HO-CH₂-***CH₂***-N-Ar₂); 3.75-3.55 (m, 6H, 2 x CH₃-***CH₂***-N-Ar and HO-***CH₂***-CH₂-N-Ar₂); 1.77 (br s, 6H, ***CH₃***-Ar); 1.43-1.29 (m, 3H, ***CH₃***-CH₂-O); 1.27-1.11 (m, 6H, ***CH₃***-CH₂-N).

### Synthesis of 2-Methoxy-ethyl-BBG

2-methoxy-ethyl-BBG was synthetized by following the general synthesis procedure reported above, using 2-Bromoethyl methyl ether as alkylating bromide (alkylating agent).

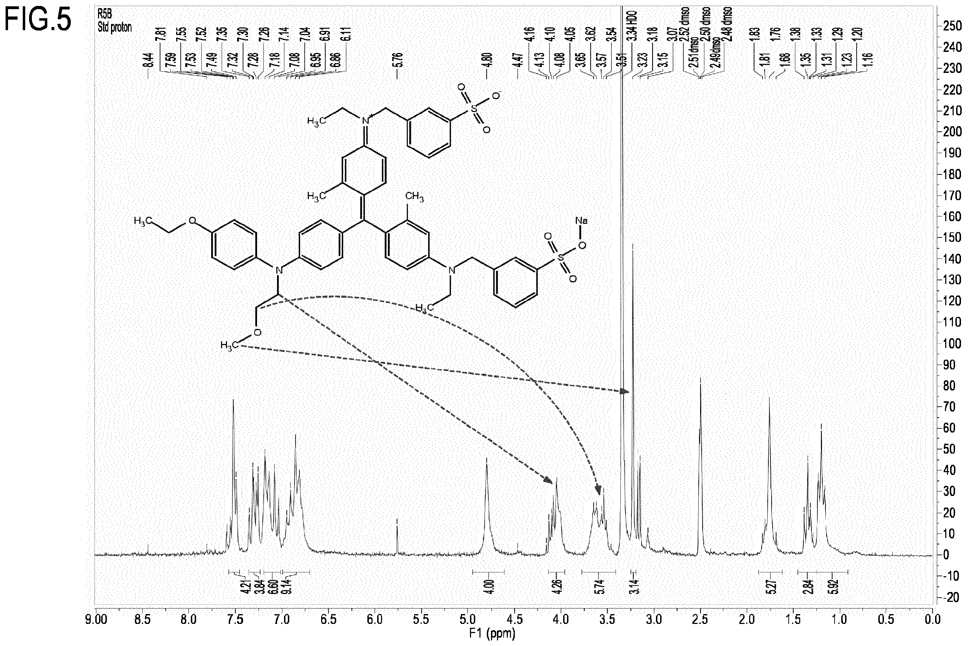

The 2-methoxy-ethyl-BBG was obtained in a reaction yield of 80% (determined by HPLC) and, after purification by flash-chromatography on silica gel using as eluent gradient of CH₂Cl₂/MeOH, it was analyzed by means of 1H-NMR on a Varian Inova 200 MHz spectrometer using CD3OD or DMSO-d6 as solvent. Figure 5 shows the 1H-NMR spectrum of the product obtained, in which the following peak attributions were made: : ¹H-NMR (DMSO-d6) d ppm: 7.60-7.44 (m, 4H, Ar***H***); 7.39-7.01 (m, 10H, Ar***H***); 7.01-6.70 (m, 8H, Ar***H***); 4.80 (br s, 2H, Ar-***CH₂***-N); 4.18-3.93 (m, 4H, CH₃-***CH₂***-O and CH₃-O-CH₂-***CH₂***-N-Ar₂); 3.76-3.44 (m, 6H, 2 x CH₃-***CH₂***-N-Ar and CH₃O-***CH₂***-CH₂-N-Ar₂); 3.23 (s, 1H, ***CH₃***-O-CH₂-CH₂-N-Ar₂)1.77 (br s, 6H, ***CH₃***-Ar); 1.40-1.27 (m, 3H, ***CH₃***-CH₂-O); 1.27-1.09 (m, 6H, ***CH₃***-CH₂-N).

From the analysis of the results of Example 2 and of Example 1, it clearly results that the process according to the present invention allows to obtain dye molecules having structure (II) with reaction times significantly shorter and with a level of purity higher than the methods of synthesis known in the prior art that do not use microwave radiation. In this manner it is possible to achieve a high productivity and at the same time to eliminate or at least markedly reduce the need of expensive further purification treatments.

### EXAMPLE 3 (invention) - Synthesis of a dye molecule according to the invention having structure (I) wherein R₁ is benzyl without microwaves radiation

In a glass reactor of 250 ml equipped with a dropping funnel and a magnetic stirrer, 3.04 mmol (2.60 g) of BBG (Sigma-Aldrich 90% purity), 60.8 mmol of benzyl-bromide (7.22 ml), 60.8 mmol of NaOH (2.43 g) and 100 mg of KI (0.60 mmol) were added to 50 ml of 1/1 v/v methanol/water mixture. The mixture thus obtained was stirred at room temperature.

A sample of the reaction mixture was withdrawn from the reactor every 20 minutes and analyzed by HPLC (by using an Agilent 1260 Infinity II HPLC instrument, equipped with a Diode Array Detector Agilent G7115A as detector and using a ternary eluent consisting of methanol, acetonitrile and triethylamine 0.3% at pH 3.0 with HCOOH and a column Eurosphere II 100-3 C18 from Knauer) for detecting the formation of the target compound (Benzyl-BBG) and the reaction was continued for 90 minutes until complete conversion of the BBG of the starting mixture.

At the end of the reaction, an overall reaction yield of 90% (determined by HPLC) at 95% of purity for Benzyl-BBG was obtained.

### EXAMPLE 4 (invention) - Synthesis of a dye molecule according to the invention having structure (I) wherein R₁ is benzyl with a method according to the invention including a microwaves radiation

Microwave-assisted reactions were carried out in a CEM Discover System 0.04 mmol (33 mg) of BBG (Sigma-Aldrich 90% purity), 1.2 mmol of benzyl-bromide (0.142 ml), 1.2 mmol of NaOH (48 mg) and 0.18 mmol of KI (30 mg) were added to 4 ml of 1/1 v/v methanol/water mixture. The mixture thus obtained was irradiated at 80°C with 150 W of a microwave radiation at 2455 MHz.

The reaction was completed, i.e. a complete conversion of the BBG of the starting mixture was observed, after 10 minutes starting from the beginning of the irradiation.

At the end of the reaction, an overall reaction yield of 95% (determined by HPLC) at 99% of purity for Benzyl-BBG was obtained.

The Benzyl-BBG thus obtained was analyzed by means of 1H-NMR on a Varian Inova 200 MHz spectrometer using DMSO-d6 as solvent.

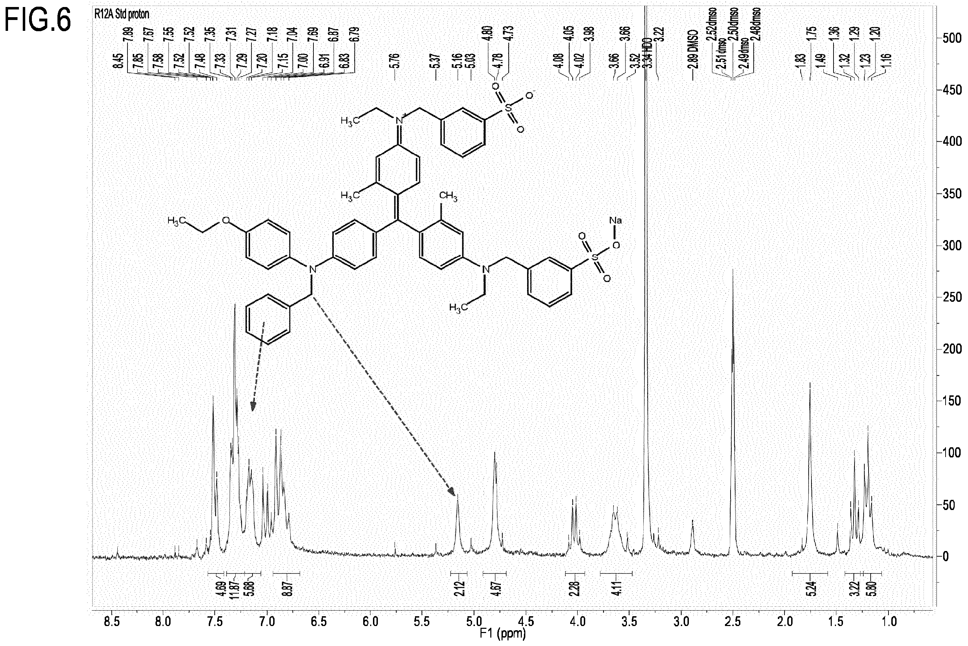

Figure 6 shows the 1H-NMR spectrum of the product obtained, in which the following peak attributions were made: ¹H-NMR (DMSO-d6) d ppm: 7.57-7.43 (m, 4H, Ar***H***); 7.42-7.09 (m, 15H, Ar***H***); 6.94-6.67 (m, 8H, Ar***H***); 5.16 (br s, 2H Ar-***CH₂***-N-Ar₂); 4.78 (br s, 2H, Ar-***CH₂***-N); 4.13-3.99 (m, 2H, ***CH₃***-CH₂-O); 3.96-3.79 (m, 2H, CH₃-***CH₂***-N-Ar₂); 3.74-3.52 (m, 4H, CH₃-***CH₂***-N-Ar); 1.75 (br s, 6H, ***CH₃***-Ar); 1.40-1.28 (m, 3H, ***CH₃***-CH₂-O); 1.27-1.08 (m, 9H, ***CH₃***-CH₂-N).

The Benzyl-BBG thus obtained was also analyzed by means of RP-HPLC (by using an Agilent 1260 Infinity II HPLC instrument, equipped with a Diode Array Detector Agilent G7115A as detector and using a ternary eluent consisting of methanol, acetonitrile and triethylamine 0.3% at pH 3.0 with HCOOH and a column Eurosphere II 100-3 C18 from Knauer).

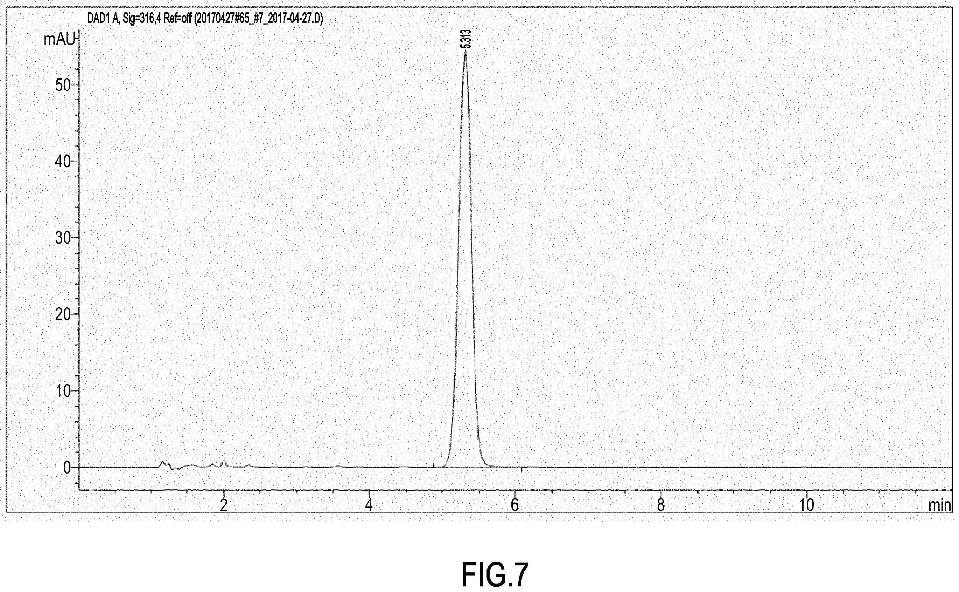

Figure 7 shows the RP-HPLC chromatogram of the product obtained, showing at an elution time of 5.313 minutes the peak of the Benzyl-BBG.

From the analysis of the results of Example 4 and of Example 3, it clearly results that the process according to the present invention allows to obtain dye molecules having structure (II) with reaction times significantly shorter and with a level of purity higher than the methods of synthesis known in the prior art that do not use microwave radiation, thereby achieving a high productivity and at the same time eliminating or at least markedly reducing the need of expensive further purification treatments.

### EXAMPLE 5 (not according to the invention) - Synthesis of a dye molecule having structure (I) wherein R₁ is methyl with a method including a microwaves radiation

Microwave-assisted reactions were carried out in a CEM Discover System 0.06 mmol (50 mg) of BBG (Sigma-Aldrich 90% purity), 0.30 mmol of methyl-iodide (19 µl), 0.15 mmol of NaOH (6 mg) were added to 4 ml of 1/1 v/v methanol/water mixture. The mixture thus obtained was irradiated at 80°C with 150 W of a microwave radiation at 2455 MHz while adding a further amount of 1.5 mmol of methyl-iodide (95 µl), 0.75 mmol of NaOH (30 mg).

Said additional methyl-iodide and NaOH were added to the reaction mixture in several aliquots during the reaction at time intervals of 5 minutes starting from the beginning of the irradiation as shown in Table 3.

A sample of the reaction mixture was withdrawn from the reactor every 5 minutes and analyzed by HPLC (by using an Agilent 1260 Infinity II HPLC instrument, equipped with a Diode Array Detector Agilent G7115A as detector and using a ternary eluent consisting of methanol, acetonitrile and triethylamine 0.3% at pH 3.0 with HCOOH and a column Eurosphere II 100-3 C18 from Knauer) for detecting the formation of the target compound (Methyl-BBG) and the reaction was continued for 30 min starting from the beginning of the irradiation, until complete conversion of the BBG of the starting mixture.

**Table 3**

| **Time from the beginning of the irradiation (min)** | **Methyl-iodide (mmol)** | **NaOH (mmol)** |
|---|---|---|
| 5 | 0.30 | 0.15 |
| 10 | 0.30 | 0.15 |
| 15 | 0.30 | 0.15 |
| 20 | 0.30 | 0.15 |
| 25 | 0.30 | 0.15 |
| 30 | | |

At the end of the reaction, an overall reaction yield of 97% (determined by HPLC) at 99% of purity for Methyl-BBG was obtained.

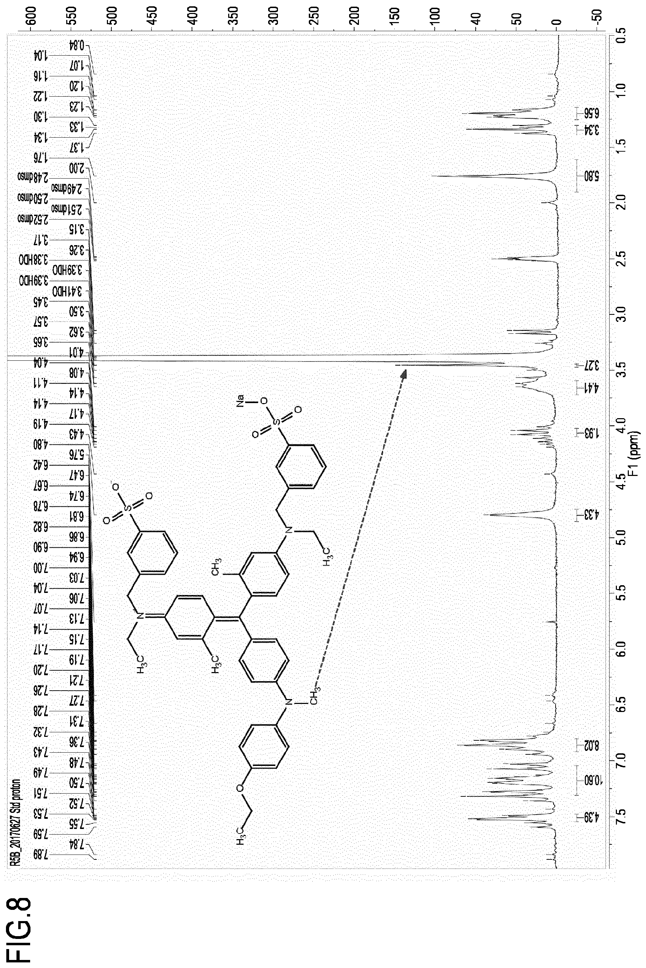

The Methyl-BBG thus obtained was analyzed by means of 1H-NMR NMR on a Varian Inova 200 MHz spectrometer using DMSO-d6 as solvent. Figure 8 shows the 1H-NMR spectrum of the product obtained, in which the following peak attributions were made: ¹H-NMR (DMSO-d6) d ppm: 7.63-7.47 (m, 4H, Ar***H***); 7.39-7.00 (m, 10H, Ar***H***); 6.94-6.73 (m, 8H, Ar***H***); 4.80 (br s, 2H, Ar-***CH₂***-N); 4.12-3.99 (m, 2H, CH₃-***CH₂***-O); 3.74-3.55 (m, 4H, CH₃-***CH₂***-N-Ar); 3.46 (s, 3H, ***CH₃***-N-Ar₂);1.76 (br s, 6H, ***CH₃***-Ar); 1.40-1.27 (m, 3H, ***CH₃***-CH₂-O); 1.27-1.13 (m, 6H, ***CH₃***-CH₂-N).

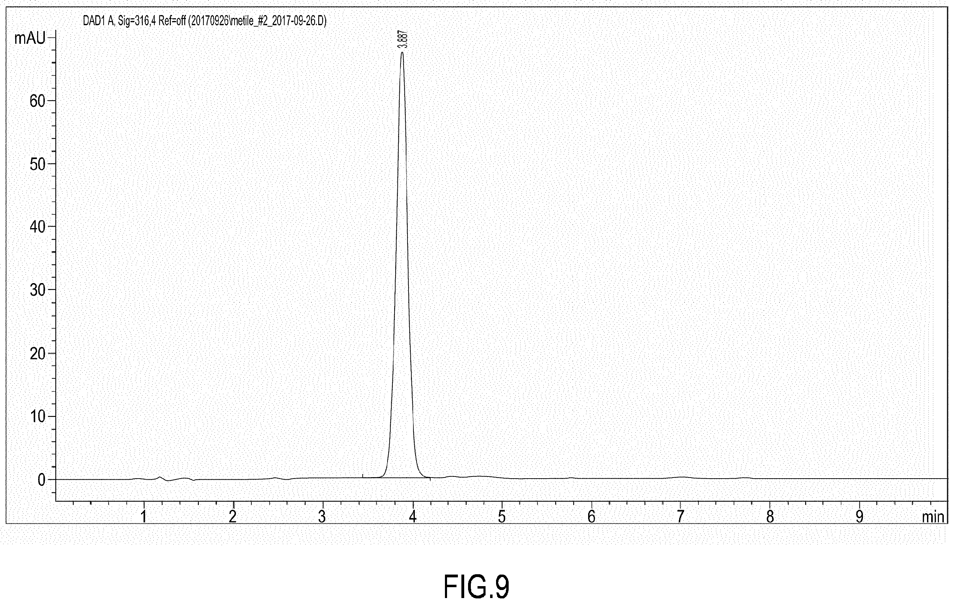

The Methyl-BBG thus obtained was also analyzed by means of RP-HPLC (by using an Agilent 1260 Infinity II HPLC instrument, equipped with a Diode Array Detector Agilent G7115A as detector and using a ternary eluent consisting of methanol, acetonitrile and triethylamine 0.3% at pH 3.0 with HCOOH and a column Eurosphere II 100-3 C18 from Knauer) . Figure 9 shows the RP-HPLC chromatogram of the product obtained, showing at an elution time of 3.887 min. of the peak of the Methyl-BBG.

### EXAMPLE 6 - Protein assay using the Benzyl-BBG according to the invention, the Ethyl-BBG according to Example 2, compared with BBG as reference staining agent

The dye molecules according to the invention were tested in order to show their higher selectivity for proteins and protein chains with respect to BBG. For this purpose, SDS-PAGE electrophoresis tests were performed in order to compare the Benzyl-BBG according to the invention (Example 4) and the Ethyl-BBG according to Example 2 with a commercially available grade of BBG (Sigma-Aldrich 90% purity).

A set of molecular weight standards was created by mixing different amounts of known proteins (E.coli beta-galactosidase - 116.3 kDa, bovine serum albumin - 66.5 kDa, chicken ovalbumin - 42.7 kDa and equine cytochrome C - 12,384 Da) in such a way as to obtain bands of similar intensity. In order to do so, an inverse relationship between amount (as nanograms of protein) and molecular weight of any protein was initially achieved, and small adjustments were experimentally made. Polyacrylamide gel electrophoresis was done in 9% precast gels (Bio-Rad) at 20V for 60-90 minutes in denaturing conditions; standards were loaded in triplicate on separate lanes. After the completion of the run, each lane was cut off and soaked overnight in a different staining solution, consisting either the reference staining agent ("BBG", Sigma-Aldrich 90% purity), the dye molecule according to Example 4 Benzyl-BBG or the dye molecule according to Example 2 Ethyl-BBG dissolved at 0.05% by weight/volume in Gel fixative solution (40% methanol, 20% acetic acid). Gel lanes were decolored in the above fixative solution without stain until the background stain reached the least level, and images were taken through a manual scanner at 300 dpi, 16M colors. Densitometric analysis was done using the software ImageJ (NIH.gov).

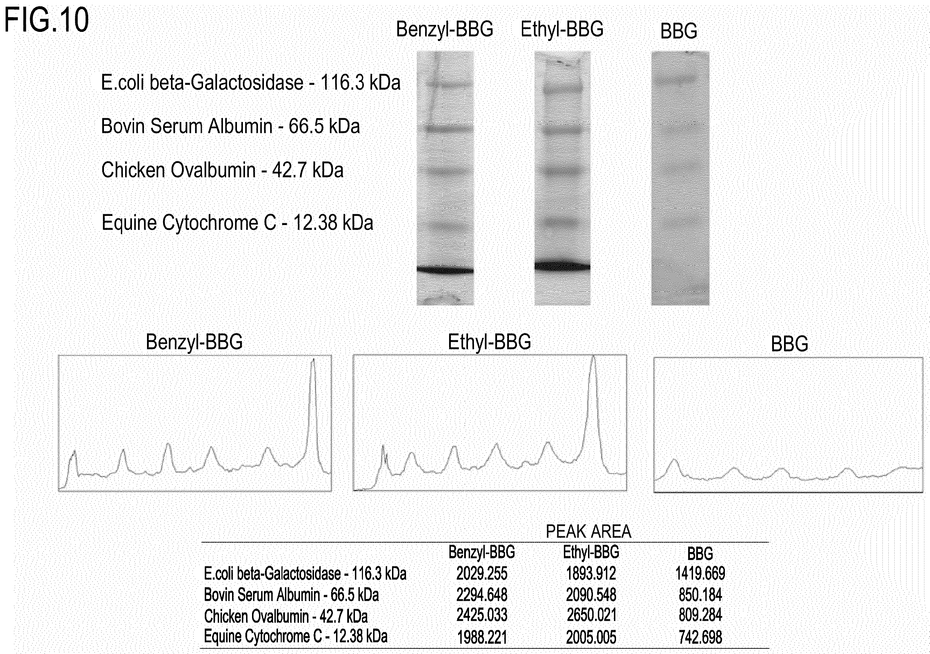

The results obtained are shown in figure 10, and showed that gel treated with the dyes according to the invention give more intense spots with a clear background. In particular, the reduced staining of the background with respect to the reference BBG made the spots of the analyzed proteins more visible and better quantifiable by densitometric analysis.

In addition, it was also observed that the spot intensities of the analyzed proteins obtained from Benzyl-BBG and Ethyl-BBG were significantly higher (p <0.05) with respect to BBG. In fact, for beta-galactosidase, bovine serum albumin, chicken ovalbumin and equine cytochrome C, there was an increase in the area counts, with respect to BBG, of 38%, 157%, 213% and 168%, respectively. No significant difference was finally observed between the spot intensity of Benzyl-BBG and Ethyl-BBG.

### EXAMPLE 7 - Preparation of three staining compositions comprising Benzyl-BBG according to the invention

Three staining compositions in the form of a solution according to the invention was prepared by mixing the Benzyl-BBG according to the invention (Example 4) and lutein with other conventional ingredients according to a method known to a person skilled in the art. In this way, three staining compositions were obtained having the compositions reported in the following Table 4.

**Table 4**

| **Ingredient** | **Composition A** | **Composition B** | **Composition C** |
|---|---|---|---|
| Lutein 1 (% by weight) | - | - | 1.8 |
| Lutein 2 (% by weight) | 2 | 2 | - |
| Benzyl-BBG according to Example 4 (% by weight) | 0.05 | 0.05 | 0.05 |
| Trypan Blue (% by weight) | 0 | 0.15 | 0.15 |
| Sodium chloride (% by weight) | 0.400 | 0.400 | 0.400 |
| Sodium hydrogen phosphate dihydrate (% by weight) | 0.711 | 0.711 | 0.711 |
| Sodium dihydrogen phosphate dihydrate (% by weight) | 0.368 | 0.368 | 0.368 |
| Polyvinylpyrrolidone (Plasdone PVPK29/32) | 0 | 0 | 0.400. |
| Polyvinyl alcohol | 0 | 0 | 1.400 |
| Water (% by weight) | up to 100% | up to 100% | up to 100% |
| Legend: | | | |
| Lutein 1: FloraGLO Crystals (Kemin); | | | |
| Lutein 2: FloraGLO Lutein 5% CWS/S-TG (Royal DSM); | | | |
| Trypan Blue: Sigma Aldrich; | | | |
| Sodium chloride: Sigma Aldrich; | | | |
| Sodium hydrogen phosphate dihydrate: Sigma Aldrich; | | | |
| Sodium dihydrogen phosphate dihydrate: Sigma Aldrich; | | | |
| Plasdone PVPK29/32: Sigma Aldrich; | | | |
| Polyvinyl alcohol: Sigma Aldrich. | | | |

### EXAMPLE 8 - Dyeing test of three staining compositions comprising BBG, methyl-BBG according to WO 2016/189475 and Benzyl-BBG according to the invention

The benzyl-BBG dye molecule according to the invention was tested in order to show its higher selectivity in the dyeing of the ILM with respect to BBG and methyl-BBG according to WO 2016/189475.

To this end, a staining composition according to the invention was prepared having the following formulation (% by weight).
- dye: Benzyl-BBG according to Example 4: 0.025%
- Sodium phosphate monobasic dihydrate: 0.368% (SIGMA ALDRICH)
- Sodium phosphate bibasic dihydrate: 0.711% (SIGMA ALDRICH)
- Sodium chloride: 0.400% (SIGMA ALDRICH)
- water for injectables: up to 100% (FRESENIUS KABI ITALIA S.R.L.).

This staining composition was compared with two staining composition available on the market, more specifically:
- a staining composition including BBG and marketed under the trade name of ILM-BLUE^{®} (D.O.R.C. Dutch Ophthalmic Research Center (International) B.V.), and with
- a staining composition including methyl-BBG according to WO 2016/189475 marketed under the trade name of VIEW-ILM (AL.CHI.MI.A. S.r.l.).

The formulations of these comparative compositions, as declared by the manufacturers in the products analyzed, is the following (% by weight).

### ILM-BLUE^{®} (D.O.R.C.)

- dye: Brilliant Blue: G 0.025%
- disodium hydrogen phosphate dodecahydrate: 0.31%
- sodium dihydrogen phosphate dihydrate: 0.03%
- sodium chloride: 0.82%
- PEG: 4%
- water for injectables: up to 100%.

### VIEW ILM (ALCHIMIA)

- dye: methyl-BBG: 0.03%
- thickening agent (Ficoll^{®} 400 at 4% in physiologic solution)
- sodium chloride
- sodium phosphate monobasic
- sodium phosphate bibasic
- water for injectables: up to 100%.

### Ex-vivo staining procedure

Porcine eyes were obtained from a slaughterhouse and were prepared within 5 hours after death, the eyes were placed on a plastic holder, and a 360-degree limbal incision was performed for corneal removal, followed by total iris removal. Whole vitreous was gently extracted.

Next, 1 ml of BSS was placed in the eye and 40 µl of dye formulation was gently placed in the eye, by a Gilson pipet, for 30 seconds; then the BSS along the dye were removed and the eye rinsed with BSS.

The peeling of ILM was carried out by forceps and the tissue placed in an Eppendorf tube, the rest of retina near the area of ILM peeling was collected and placed in another Eppendorf tube.

The average amount of ILM tissue was of about 2 mg, whereas the average amount of retina tissue was of about 20 mg.

All tissues (ILM and retina) were stored at -20°C before HPLC analysis.

### Dye extraction and analysis

Retina and ILM tissue samples obtained as described above were weighed and homogenized in 250-500 µl of methanol. The homogenates were then transferred in an Eppendorf tube, ultrasonically extracted using a Branson Sonifier, centrifuged at 1,000 x g for 10 min and filtered on 0.45 µm syringe filters. The filtrates were transferred into labeled, clean test tubes and evaporated to dryness under a stream of nitrogen. The residues were reconstituted in 100 µl of mobile phase by vortexing for 120 s, filtered again on 0.45 µm filters and, finally, 20 µl aliquots were injected onto the chromatographic apparatus (each sample was analyzed in triplicate).

HPLC analyses were performed on an Agilent 1260 Infinity II chromatographic system (Agilent Technologies) equipped with a ChemStation OpenLab software, a quaternary pump, a diode array detector (DAD) and a thermostated column compartment. Chromatographic separations were performed using a ternary eluent consisting of methanol, acetonitrile and triethylamine 0.3% at pH 3.0 with HCOOH with a column Eurosphere II 100-3 C18 from Knauer.

Identification of the dye molecule (BBG, methyl-BBG and Benzyl-BBG) was performed by HPLC-DAD analysis by comparing the retention time and the UV spectra of the samples with those of authentic reference samples. Potential interferences from endogenous matrix constituents of ILM and retina tissues were unequivocally excluded by analyzing the chromatograms of blank tissue samples. In addition, peak-purity tests using photodiode-array detector spectra were used to show that the analyte peaks cannot be attributed to more than one component.

The peak purity indexes were evaluated by OpenLab software (Agilent Technologies) and were found to be >990. Quantification of the dye molecule was performed using 6-10 points external regression curves performed at 613 nm. The calibration curves obtained gave correlation coefficients higher than 0.99. Method validation was performed according to the principles of FDA (Bioanalytical Method Validation Guidance for Industry, release 05/24/18) and EMEA (Guideline on bioanalytical method validation, 21 July 2011, EMEA/CHMP/EWP/192217/2009 Rev. 1 Corr. 2**) for bioanalytical method validation.

### Results

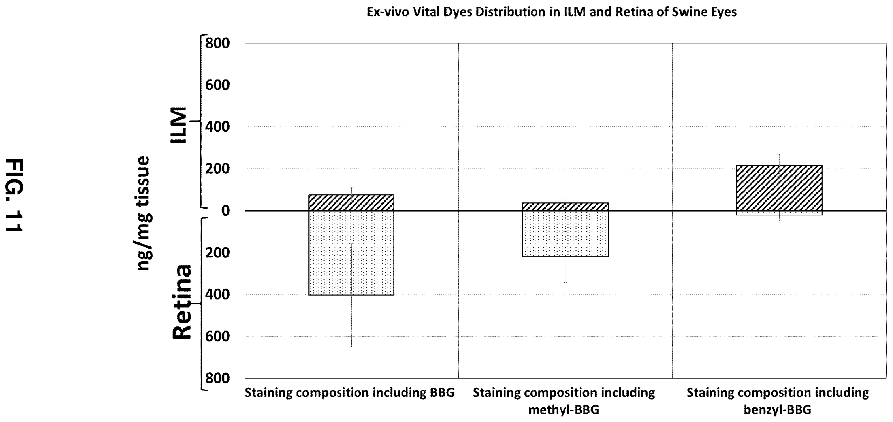

The amounts of the dye molecule in the ILM and retina (mean values of 6 measurements and standard deviation) measured in the tests are summarized in Table 5 hereinbelow. These data are also graphically shown in the diagram of Fig. 11, which indicates the dye content in ILM and retina tissues of the swine eyes treated with the above staining compositions.

In the data analysis, statistical differences of *ex-vivo* data are determined using analysis of variance (ANOVA) followed by the Bonferroni-Dunn post hoc pairwise comparison procedure. A probability, P, of less than 0.05 was considered significant (n=6 eyes).

**Table 5**

| **Tested structure** | **Reference composition including BBG** | **Reference composition including methyl-BBG** | | **Composition according to the invention including** | **Benzyl-BBG according to Example 4** | |
|---|---|---|---|---|---|---|
| | mean | sd | mean | sd | mean | sd |
| ILM | 74.26 | 35.64 | 36.47 | 22.77 | 214.88 | 53.79 |
| Retina | 402.50 | 246.92 | 219.18 | 123.80 | 19.69 | 37.37 |

As may be gathered by the above experimental results, the staining formulation including the benzyl-BBG derivative according to the invention achieves a significant increase of the dye content in the ILM tissue with respect to the comparative formulations of the prior art including BBG and methyl-BBG respectively.

The dye levels detected in the group of eyes treated *ex-vivo* with the staining formulation according to the invention (about 215 ng/mg tissue of benzyl-BBG derivative) was significantly higher with respect to the groups of eyes treated with the known formulations including BBG (about 74 ng/mg tissue, p = 0,002) and methyl-BBG (about 36 ng/mg tissue, p<0,001), with an increase of dye content of 2.9- and 6.0-fold, respectively.

The improvement with respect to the formulation including the methyl-BBG derivative according to WO 2016/189475 is particularly significant, since a lower amount of the dye molecule of the invention has been used in the tests (0.025% by weight of benzyl-BBG vs. 0.030% by weight of methyl-BBG).

Quite advantageously, the staining formulation including the benzyl-BBG derivative according to the invention showed at the same time a very low retinal migration of the dye compared to the reference formulations according to the prior art and including BBG and methyl-BBG.

Actually, the dye content identified in the retina of the groups treated with BBG (about 402 ng/mg tissue) and methyl-BBG (about 219 ng/mg tissue) were significantly higher (p=0.004 for both formulations) respect to the staining formulation according to the invention (about 20 ng/mg tissue), with a 20.1- and 10.9-fold increase in the dyes content, respectively.

The improved staining selectivity achieved by the dye molecules and by the staining compositions of the present invention as compared to the staining compositions of the prior art most advantageously enhances the color contrast between the intraocular membranes of interest and the retina, thereby significantly reducing the risk that surgical vitreoretinal procedures may cause a mechanical damage to the retina.

Also, the highly selective staining of the ILM achieved by the dye molecules and by the staining compositions of the present invention significantly reduces the risk of possible toxic effects on the retinal tissue with the same dye concentration in the staining formulation, opening at the same time the possibility of increasing the dye concentration, with an enhancement of the staining effect, while still operating in safe conditions from a toxicological point of view.

## Claims

1. Dye molecule having structure (I) 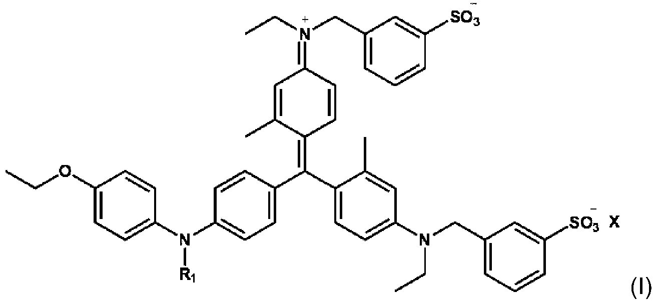 wherein
X represents an organic or inorganic cation; and
R₁ represents a substituted or unsubstituted benzyl group selected from benzyl, 4-methylbenzyl, 2-methylbenzyl, 3-methylbenzyl, 3,5-dimethylbenzyl, 4-chlorobenzyl, 4-isopropylbenzyl, 2-(methyl)-1,3-dimethylbenzene, 4-(methyl)benzonitrile, 4-(trifluoromethyl)benzyl, 2,6-dichlorobenzyl, 2-chlorobenzyl, 1-(methyl)-2,3-dimethylbenzene, 3,4-dichlorobenzyl, 3,5-di-tert-butylbenzyl, 3-(methyl)benzonitrile, 4-fluorobenzyl, 1-(methyl)-3-(difluoromethyl)benzene, 3-(trifluoromethyl)benzyl, 4-bromobenzyl, 4-(methyl)benzoic acid, 2,3-dichlorobenzyl, 2,5-dichlorobenzyl, 4-iodobenzyl, 3-fluorobenzyl, 3,5-bis(trifluoromethyl)benzyl, 1-(methyl)-2-(difluoromethyl)benzene, 1-(methyl)-2,4-dimethylbenzene, 4-(methyl)-3-methylbenzonitrile, 4-methyl-benzamide, 4-(methylthio)benzyl, 2-(methyl)benzyl alcohol, 2-(trifluoromethyl)benzyl, 2-fluorobenzyl, 3-bromobenzyl, 2-bromobenzyl, 3,5-difluorobenzyl, 4-tert-butylbenzyl, 3-chlorobenzyl, 2-(methyl)benzonitrile, 1-methyl-4-(2,2,2-trifluoroethyl)benzene, 4-(methyl)-N,N-dimethylaniline hydrobromide, 2-iodobenzyl, 2,6-difluorobenzyl, 3-iodobenzyl, 3,5-dibromobenzyl, 3-(methyl)benzyl methyl ether, 3-(methyl)benzoic acid, 1-(methyl)-3-chloro-5-fluorobenzene, 3-fluoro-5-methylbenzyl, 2-(methyl)-1-methyl-4-(trifluoromethyl)benzene, 3-fluoro-4-methylbenzyl, 4-bromo-1-(methyl)-2-chlorobenzene, 4-fluoro-2-methylbenzyl, 4-phenoxybenzyl, 4-chloro-3-fluorobenzyl, 1-bromo-2-(methyl)-3-chlorobenzene, 2-fluoro-4-methylbenzyl, 4-fluoro-3-methylbenzyl, 2-[4-(methyl)phenyl]propionic acid, 2-chloro-6-fluorobenzyl, 4-(methyl)phenylboronic acid, 3-methoxybenzyl, 2,4,6-trifluorobenzyl, 2,4-difluorobenzyl, 3,4-difluorobenzyl, 4-(methyl)benzophenone, 2-methyl-3-(trifluoromethyl)benzyl, 2-chloro-4-fluorobenzyl, methyl 4-(methyl)benzoate, 4-chloro-2-fluorobenzyl, 2-fluoro-3-methylbenzyl, 3,5-dimethoxybenzyl, 1-bromo-4-(methyl)-2-chlorobenzene, 5-methyl-2-(trifluoromethyl)benzyl, 4-chloro-2,6-difluorobenzyl, 4-(methyl)-2-chloro-1-fluorobenzene, 5-fluoro-2-methylbenzyl, 3,4,5-trifluorobenzyl, 2,5-difluorobenzyl, 4-nitrobenzyl, 2-(methyl)-1-fluoro-3-methylbenzene, 2-fluoro-5-methylbenzyl, 3-chloro-5-(trifluoromethyl)benzyl, 2-chloro-5-fluorobenzyl, 4-(difluoromethoxy)benzyl, 2,3-difluorobenzyl, 3-chloro-2-fluorobenzyl, 2-[2-(methyl)phenyl]-2-methylpropanenitrile, 2-chloro-4-methoxybenzyl, ethyl 4-(methyl)benzoate, 2,3-difluoro-4-methylbenzyl, 2,6-difluoro-3-methylbenzyl, 4-chloro-3-(trifluoromethyl)benzyl, 5-chloro-2-fluorobenzyl, 4-(trifluoromethylthio)benzyl, 2-chloro-5-(trifluoromethyl)benzyl, 4-(methylsulfonyl)benzyl, methyl 3-(methyl)benzoate, 4-(trifluoromethoxy)benzyl, 3-bromo-5-fluorobenzyl, 4-chloro-2-(trifluoromethyl)benzyl, 3-phenylbenzyl, 2,3,5,6-tetrafluorobenzyl, 6-chloro-2-fluoro-3-methylbenzyl, 2-methyl-3-(trifluoromethyl)benzyl, 4-phenoxybenzyl, 2,3,5-trifluorobenzyl, 2,3,6-trifluorobenzyl, 3-nitrobenzyl, 3-(trifluoromethoxy)benzyl, [4-(methyl)phenoxy]acetonitrile, 2-bromo-1-(methyl)-4-tert-butylbenzene, 1-bromo-4-(methyl)-2-fluorobenzene, 2-bromo-4-fluorobenzyl, 2-chloro-6-(trifluoromethyl)benzyl, 1-(methyl)-2-phenoxybenzene, 4-cyano-2-fluorobenzyl, 3-chloro-2,4-difluorobenzyl, 4-(methyl)-2-fluorobenzonitrile, 5-bromo-2-(methyl)-1,3-difluorobenzene, 4-methyl-2,3,5,6-tetrafluorobenzyl, 2,4-dichloro-5-fluorobenzyl, 2-chloro-6-fluoro-3-methylbenzyl, 2-chloro-3,6-difluorobenzyl, 5-chloro-2-(trifluoromethyl)benzyl, 3-chloro-2,6-difluorobenzyl, 2,4,5-trifluorobenzyl, 2-phenylbenzyl, 3-fluoro-5-(trifluoromethyl)benzyl, 3-fluoro-4-(trifluoromethyl)benzyl, 3-(difluoromethoxy)benzyl, 4-bromo-2-fluorobenzyl, 2-bromo-4-(methyl)-1-fluorobenzene, 2,3,4-trifluorobenzyl, 2-bromo-1-(methyl)-3-fluorobenzene, 1-(methyl)-3-(methylsulfonyl)benzene, 4-bromo-3-(methyl)benzonitrile, 3-methyl-4-nitrobenzyl, 3-cyano-4-fluorobenzyl, tert-butyl 3-(methyl)benzoate, 2-bromo-5-fluorobenzyl, 2-fluoro-4-(trifluoromethyl)benzyl, 2-fluoro-3-(trifluoromethyl)benzyl, 2,3,4,5,6-pentafluorobenzyl, 4-bromo-1-(methyl)-2-ethylbenzene, 2-bromo-1-(methyl)-3-(trifluoromethyl)benzene, 2-(methyl)-4-chloro-1-methoxybenzene, 3-bromo-2-fluorobenzyl, 1-(methyl)-2-methyl-4-(trifluoromethoxy)benzene, 4'-methyl-2-biphenylcarbonitrile, 4-fluoro-3-(trifluoromethyl)benzyl, 2-fluoro-5-(trifluoromethyl)benzyl, 2-nitrobenzyl, 4-(methyl)-2-methoxybenzonitrile, 2-(methyl)-5-fluorobenzonitrile, 1-(methyl)-3-methyl-5-(trifluoromethoxy)benzene, 5-fluoro-2-(trifluoromethyl)benzyl, 1-(methyl)-2-(difluoromethoxy)benzene, 4-fluoro-2-(trifluoromethyl)benzyl, 4-(methyl)-3-methoxybenzonitrile, 4-methoxy-3-(trifluoromethyl)benzyl, 3-fluoro-4-methoxybenzyl, 2-(trifluoromethoxy)benzyl, 3-(methyl)-4-methoxybenzonitrile, 3-bromo-2-(methyl)benzonitrile, 4-bromo-1-(methyl)-2-(trifluoromethyl)benzene, methyl 3-(methyl)-4-chlorobenzoate, 2-(methyl)-3-fluorobenzonitrile, 1-(methyl)-3-(4-fluorophenoxy)benzene, 2-(methyl)-1-fluoro-4-methoxybenzene, 2-bromo-6-(trifluoromethyl)benzyl, 4-fluoro-3-methoxybenzyl, 5-fluoro-2-methoxybenzyl, 3-fluoro-5-methoxybenzyl, 2-bromo-5-methoxybenzyl, 2,3,5,6-tetrafluoro-4-(trifluoromethyl)benzyl, phenacyl 4-(methyl)phenylacetate, 2-fluoro-6-(trifluoromethyl)benzyl, 2-hydroxy-5-nitrobenzyl, 2-fluoro-3-methoxybenzyl, 4-methyl-N,N-dimethyl-benzenesulfonamide, 4-bromo-2-(methyl)-1-methoxybenzene, tert-butyl [2-(methyl)phenoxy]acetate, 4-(methyl)-1-ethoxy-2-(trifluoromethyl)benzene, 4-chloro-3-(trifluoromethoxy)benzyl, 2-fluoro-6-methoxybenzyl, 3-fluoro-4-nitrobenzyl, 2-bromo-1-methyl-5-methoxy-3-methylbenzene, methyl 4-(methyl)-3-methoxybenzoate, 1-bromo-2-(methyl)-4,5-dimethoxybenzene, (4-(methyl)phenyl)(4-(prop-2-yn-1-yloxy)phenyl)methanone, (4-(methyl)phenyl)(4-(prop-2-yn-1-yloxy)phenyl)methanone, tert-butyl 2,4-difluoro-3-methoxybenzyl, 2,6-difluoro-3-methoxybenzyl, 2-nitro-4-(trifluoromethyl)benzyl, 4-fluoro-3-nitrobenzyl, 4-methyl-3-nitrobenzoic acid, methyl 3-bromo-5-(methyl)benzoate, 2-fluoro-5-(trifluoromethoxy)benzyl, methyl 2-bromo-4-(methyl)benzoate, methyl 3-(methyl)-2-methoxybenzoate, 4-bromo-1-(methyl)-2-(methylsulfonyl)benzene, methyl 4-bromo-2-(methyl)benzoate, 4-fluoro-2-nitrobenzyl, 4-bromo-1-(methyl)-2-nitrobenzene, 2-[(phenylsulfonyl)methyl]benzyl, ethyl 4-bromo-2-(methyl)benzoate, methyl 2-(methyl)-4-nitrobenzoate, 1-(methyl)-2-fluoro-3-(trifluoromethoxy)benzene, methyl 3-bromo-2-(methyl)benzoate, 3-ethoxy-2,4-difluorobenzyl, 4,5-dimethoxy-2-nitrobenzyl, 4-[2-(methyl)phenoxy]tetrahydropyran, 1-[3-(methyl)phenyl]-1H-pyrrole, 2-[4-(methyl)phenyl]-5-methyl, 4-benzyl-acetic acid, said substituted or unsubstituted benzyl group being preferably selected from benzyl, 4-(trifluoromethyl)benzyl, 3,5-bis(trifluoromethyl)benzyl, 4-benzyl-acetic acid, 3,4-dichlorobenzyl_or 4-(tert-butyl)benzyl.

2. Dye molecule according to claim 1, wherein X represents an organic or inorganic cation selected from the group consisting of H⁺, NH₄⁺, an ion selected from the group consisting of Na⁺, Mg⁺⁺, K⁺, Ca⁺⁺, a lysinium cation or an argininium cation.

3. Staining composition comprising the dye molecule having structure (I) according to claim 1 or 2, a pharmaceutically acceptable salt, or a hydrate thereof, the composition preferably comprising from 0.0001% to 0.5% by weight of said dye molecule having structure (I), a pharmaceutically acceptable salt, or a hydrate thereof.

4. Staining composition according to claim 3, further comprising at least one density adjuster, wherein said density adjuster is preferably selected from the group consisting of D₂O, monosaccharides, disaccharides, polysaccharides, xanthophylles and polymers, wherein said density adjuster comprises more preferably at least one xanthophyll and wherein said xanthophyll is even more preferably lutein.

5. Staining composition according to claim 3 or 4, comprising from 0.01% to 0.1% by weight of the dye molecule having structure (I), a pharmaceutically acceptable salt or a hydrate thereof, from 0.5% to 3.0% by weight of lutein, from 0% to 0.25% by weight of trypan blue.

6. Staining composition according to claim any one of claims 3 to 5, for use in a method of ophthalmic surgery comprising staining an ocular membrane or structure, wherein the ocular membrane or structure preferably comprise the anterior and posterior capsule, corneal epithelium and endothelium, epiretinal membrane, internal limiting membrane, vitreous or posterior hyaloid and wherein said method of ophthalmic surgery preferably comprises the removal of an ocular membrane or structure.

7. Method of staining proteins in a sample comprising the step of contacting said sample with the staining composition according to any one of claims 3 to 5, wherein said staining is preferably performed to determine one or more analytical parameter of said proteins, said analytical parameter being preferably selected from the group consisting of molecular weight, quantity, identity.

8. Method of synthesis of a dye molecule having structure (I) as defined in any one of claims 1 or 2,
the method comprising
a. forming a reaction mixture having a pH from 8 to 14 comprising a protic solvent and a molecule having structure (III) 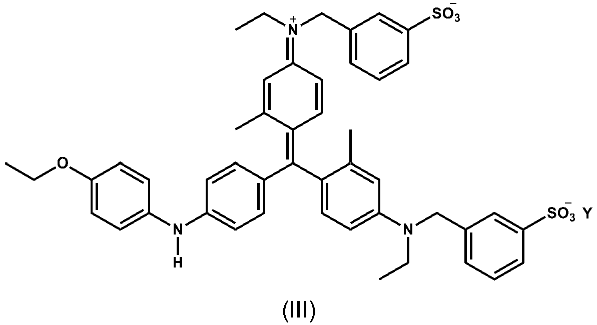 wherein Y represents an organic or inorganic cation; and
b. irradiating the reaction mixture resulting from step a. with microwave radiation comprising electromagnetic waves having power of from 5 W to 300 W in presence of an alkylating agent having structure (IV)
R₁-Z (IV)
wherein R₁ represents a substituted or unsubstituted benzyl group as defined in claim 1 and Z represents a leaving group; so as to obtain said dye molecule having structure (I), and
wherein the alkylating agent is added to the reaction mixture in one or more aliquots in the course of step b.

9. Method of synthesis according to claim 8, wherein Y represents an organic or inorganic cation selected from the group consisting of H⁺, NH₄⁺, an ion selected from the group consisting of Na⁺, Mg⁺⁺, K⁺, Ca⁺⁺, a lysinium cation or an argininium cation.

10. Method of synthesis according to any one of claims 8 or 9, wherein said leaving group Z is selected from the group consisting of halides.

11. Method of synthesis according to any one of claims 8 to 10, wherein said step b. is performed at a temperature of from 30°C to 100°C.

12. Method of synthesis according to any one of claims 8 to 10, further comprising the step of purifying said dye molecule having structure (I) resulting from step b.

## Patentansprüche

1. Farbstoffmolekül mit der Struktur (I) 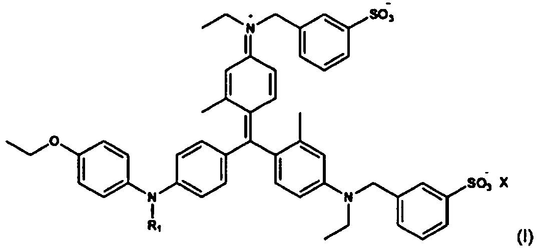 wobei
X ein organisches oder anorganisches Kation darstellt; und
R₁ eine substituierte oder unsubstituierte Benzylgruppe darstellt, ausgewählt aus Benzyl, 4-Methylbenzyl, 2-Methylbenzyl, 3-Methylbenzyl, 3,5-Dimethylbenzyl, 4-Chlorbenzyl, 4-Isopropylbenzyl, 2-(Methyl)-1,3-Dimethylbenzol, 4-(Methyl)benzonitril, 4-(Trifluormethyl)benzyl, 2,6-Dichlorbenzyl, 2-Chlorbenzyl, 1-(Methyl)-2,3-Dimethylbenzol, 3,4-Dichlorbenzyl, 3,5-Di-tert.-Butylbenzyl, 3-(Methyl)benzonitril, 4-Fluorbenzyl, 1-(Methyl)-3-(Difluormethyl)benzol, 3-(Trifluormethyl)benzyl, 4-Brombenzyl, 4-(Methyl)benzoesäure, 2,3-Dichlorbenzyl, 2,5-Dichlorbenzyl, 4-lodbenzyl, 3-Fluorbenzyl, 3,5-Bis(trifluormethyl)benzyl, 1-(Methyl)-2-(Difluormethyl)benzol, 1-(Methyl)-2,4-dimethylbenzol, 4-(Methyl)-3-methylbenzonitril, 4-Methylbenzamid, 4-(Methylthio)benzyl, 2-(Methyl)benzylalkohol, 2-(Trifluormethyl)benzyl, 2-Fluorbenzyl, 3-Brombenzyl, 2-Brombenzyl, 3,5-Difluorbenzyl, 4-tert-Butylbenzyl, 3-Chlorbenzyl, 2-(Methyl)benzonitril, 1-Methyl-4-(2,2,2-trifluorethyl)benzol, 4-(Methyl)-N,N-dimethylanilinhydrobromid, 2-Jodbenzyl, 2,6-Difluorbenzyl, 3-Jodbenzyl, 3,5-Dibrombenzyl, 3-(Methyl)benzylmethylether, 3-(Methyl)benzoesäure, 1-(Methyl)-3-chlor-5-fluorbenzol, 3-Fluor-5-methylbenzyl, 2-(Methyl)-1-methyl-4-(trifluormethyl)benzol, 3-Fluor-4-methylbenzyl, 4-Brom-1-(methyl)-2-chlorbenzol, 4-Fluor-2-methylbenzyl, 4-Phenoxybenzyl, 4-Chlor-3-fluorbenzyl, 1-Brom-2-(methyl)-3-chlorbenzol, 2-Fluor-4-methylbenzyl, 4-Fluor-3-methylbenzyl, 2-[4-(methyl)phenyl]propionsäure, 2-Chlor-6-fluorbenzyl, 4-(Methyl)phenylboronsäure, 3-Methoxybenzyl, 2,4,6-Trifluorbenzyl, 2,4-Difluorbenzyl, 3,4-Difluorbenzyl, 4-(Methyl)benzophenon, 2-Methyl-3-(trifluormethyl)benzyl, 2-Chlor-4-fluorbenzyl, Methyl-4-(methyl)benzoat, 4-Chlor-2-fluorbenzyl, 2-Fluor-3-methylbenzyl, 3,5-Dimethoxybenzyl, 1-Brom-4-(methyl)-2-chlorbenzol, 5-Methyl-2-(trifluormethyl)benzyl, 4-Chlor-2,6-difluorbenzyl, 4-(Methyl)-chlor-1-fluorbenzol, 5-Fluor-2-methylbenzyl, 3,4,5-Trifluorbenzyl, 2,5-Difluorbenzyl, 4-Nitrobenzyl, 2-(Methyl)-1-fluor-3-methylbenzol, 2-Fluor-5-methylbenzyl, 3-Chlor-5-(trifluormethyl)benzyl, 2-Chlor-5-fluorbenzyl, 4-(Difluormethoxy)benzyl, 2,3-Difluorbenzyl, 3-Chlor-2-fluorbenzyl, 2-[2-(Methyl)phenyl]-2-methylpropannitril, 2-Chlor-4-methoxybenzyl, Ethyl-4-(methyl)benzoat, 2,3-Difluor-4-methylbenzyl, 2,6-Difluor-3-methylbenzyl, 4-Chlor-3-(trifluormethyl)benzyl, 5-Chlor-2-fluorbenzyl, 4-(Trifluormethylthio)benzyl, 2-Chlor-5-(trifluormethyl)benzyl, 4-(Methylsulfonyl)benzyl, Methyl-3-(methyl)benzoat, 4-(Trifluormethoxy)benzyl, 3-Brom-5-fluorbenzyl, 4-Chlor-2-(trifluormethyl)benzyl, 3-Phenylbenzyl, 2,3,5,6-Tetrafluorbenzyl, 6-Chlor-2-fluor-3-methylbenzyl, 2-Methyl-3-(trifluormethyl)benzyl, 4-Phenoxybenzyl, 2,3,5-Trifluorbenzyl, 2,3,6-Trifluorbenzyl, 3-Nitrobenzyl, 3-(Trifluormethoxy)benzyl, [4-(Methyl)phenoxy]acetonitril, 2-Brom-1-(methyl)-4-tert-butylbenzol, 1-Brom-4-(methyl)-2-fluorbenzol, 2-Brom-4-fluorbenzyl, 2-Chlor-6-(trifluormethyl)benzyl, 1-(Methyl)-2-phenoxybenzol, 4-Cyano-2-fluorbenzyl, 3-Chlor-2,4-difluorbenzyl, 4-(Methyl)-2-fluorbenzonitril, 5-Brom-2-(methyl)-1,3-difluorbenzol, 4-Methyl-2,3,5,6-Tetrafluorbenzyl, 2,4-Dichlor-5-fluorbenzyl, 2-Chlor-6-fluor-3-methylbenzyl, 2-Chlor-3,6-difluorbenzyl, 5-Chlor-2-(trifluormethyl)benzyl, 3-Chlor-2,6-difluorbenzyl, 2,4,5-Trifluorbenzyl, 2-Phenylbenzyl, 3-Fluor-5-(trifluormethyl)benzyl, 3-Fluor-4-(trifluormethyl)benzyl, 3-(Difluormethoxy)benzyl, 4-Brom-2-fluorbenzyl, 2-Brom-4-(methyl)-1-fluorbenzol, 2,3,4-Trifluorbenzyl, 2-Brom-1-(methyl)-3-fluorbenzol, 1-(Methyl)-3-(methylsulfonyl)benzol, 4-Brom-3-(methyl)benzonitril, 3-Methyl-4-nitrobenzyl, 3-Cyano-4-fluorbenzyl, tert-Butyl-3-(methyl)benzoat, 2-Brom-5-fluorbenzyl, 2-Fluor-4-(trifluormethyl)benzyl, 2-Fluor-3-(trifluormethyl)benzyl, 2,3,4,5,6-Pentafluorbenzyl, 4-Brom-1-(methyl)-2-ethylbenzol, 2-Brom-1-(methyl)-3-(trifluormethyl)benzol, 2-(methyl)-4-chlor-1-methoxybenzol, 3-Brom-2-fluorbenzyl, 1-(Methyl)-2-methyl-4-(trifluormethoxy)benzol, 4'-Methyl-2-biphenylcarbonitril, 4-Fluor-3-(trifluormethyl)benzyl, 2-Fluor-5-(trifluormethyl)benzyl, 2-Nitrobenzyl, 4-(Methyl)-2-methoxybenzonitril, 2-(Methyl)-5-fluorbenzonitril, 1-(Methyl)-3-methyl-5-(trifluormethoxy)benzol, 5-Fluor-2-(trifluormethyl)benzyl, 1-(Methyl)-2-(difluormethoxy)benzol, 4-Fluor-2-(trifluormethyl)benzyl, 4-(Methyl)-3-methoxybenzonitril, 4-Methoxy-3-(trifluormethyl)benzyl, 3-Fluor-4-methoxybenzyl, 2-(Trifluormethoxy)benzyl, 3-(Methyl)-4-methoxybenzonitril, 3-Brom-2-(methyl)benzonitril, 4-Brom-1-(methyl)-2-(trifluormethyl)benzol, Methyl-3-(methyl)-4-chlorbenzoat, 2-(Methyl)-3-fluorbenzonitril, 1-(Methyl)-3-(4-fluorphenoxy)benzol, 2-(Methyl)-1-fluor-4-methoxybenzol, 2-Brom-6-(trifluormethyl)benzyl, 4-Fluor-3-methoxybenzyl, 5-Fluor-2-methoxybenzyl, 3-Fluor-5-methoxybenzyl, 2-Brom-5-methoxybenzyl, 2,3,5,6-Tetrafluor-4-(trifluormethyl)benzyl, Phenacyl-4-(methyl)phenylacetat, 2-Fluor-6-(trifluormethyl)benzyl, 2-Hydroxy-5-nitrobenzyl, 2-Fluor-methoxybenzyl, 4-Methyl-N,N-dimethyl-benzolsulfonamid, 4-Brom-2-(methyl)-1-methoxybenzol, tert-Butyl-[2-(methyl)phenoxy]acetat, 4-(methyl)-1-ethoxy-2-(trifluormethyl)benzol, 4-Chlor-3-(trifluormethoxy)benzyl, 2-Fluor-6-methoxybenzyl, 3-Fluor-4-nitrobenzyl, 2-Brom-1-methyl-5-methoxy-3-methylbenzol, 4-(Methyl)-3-methoxybenzoat, 1-Brom-2-(Methyl)-4,5-dimethoxybenzol, (4-(Methyl)phenyl)(4-(Prop-2-yn-1-yloxy)phenyl)methanon, (4-(Methyl)phenyl)(4-(prop-2-yn-1-yloxy)phenyl)methanon, tert-Butyl-2,4-difluor-3-methoxybenzyl, 2,6-difluor-3-methoxybenzyl, 2-Nitro-4-(trifluormethyl)benzyl, 4-Fluor-3-nitrobenzyl, 4-Methyl-3-nitrobenzoesäure, 3-Brom-5-(methyl)benzoesäuremethylester, 2-Fluor-5-(trifluormethoxy)benzylester, 2-Brom-4-(methyl)benzoesäuremethylester, 3-(methyl)-2-methoxybenzoesäuremethylester, 4-Brom-1-(methyl)-2-(methylsulfonyl)benzol, methyl-4-brom-2-(methyl)benzoat, 4-Fluor-2-nitrobenzyl, 4-Brom-1-(methyl)-2-nitrobenzol, 2-[(Phenylsulfonyl)methyl]benzyl, Ethyl-4-brom-2-(methyl)benzoat, Methyl-2-(methyl)-4-nitrobenzoat, 1-(Methyl)-2-fluor-3-(trifluormethoxy)benzol, Methyl-3-brom-2-(methyl)benzoat, 3-Ethoxy-2,4-difluorbenzyl, 4,5-Dimethoxy-2-nitrobenzyl, 4-[2-(Methyl)phenoxy]tetrahydropyran, 1-[3-(Methyl)phenyl]-1H-pyrrol, 2-[4-(Methyl)phenyl]-5-methyl, 4-Benzylessigsäure, wobei die substituierte oder unsubstituierte Benzylgruppe vorzugsweise ausgewählt ist aus Benzyl, 4-(Trifluormethyl)benzyl, 3,5-Bis(trifluormethyl)benzyl, 4-Benzylessigsäure, 3,4-Dichlorbenzyl oder 4-(tert-Butyl)benzyl.

2. Farbstoffmolekül nach Anspruch 1, wobei X ein organisches oder anorganisches Kation darstellt, ausgewählt aus der Gruppe bestehend aus H⁺, NH₄⁺, einem lon, ausgewählt aus der Gruppe bestehend aus Na⁺, Mg⁺⁺, K⁺, Ca⁺⁺, einem Lysiniumkation oder einem Argininiumkation.

3. Färbezusammensetzung, umfassend das Farbstoffmolekül mit der Struktur (I) nach Anspruch 1 oder 2, ein pharmazeutisch annehmbares Salz oder ein Hydrat davon, wobei die Zusammensetzung vorzugsweise 0,0001 bis 0,5 Gew.-% des Farbstoffmoleküls mit der Struktur (I), ein pharmazeutisch annehmbares Salz oder ein Hydrat davon umfasst.

4. Färbezusammensetzung nach Anspruch 3, ferner umfassend mindestens ein Dichteeinstellmittel, wobei das Dichteeinstellmittel vorzugsweise ausgewählt ist aus der Gruppe bestehend aus D₂O, Monosacchariden, Disacchariden, Polysacchariden, Xanthophyllen und Polymeren, wobei das Dichteeinstellmittel vorzugsweise mindestens ein Xanthophyll umfasst und wobei das Xanthophyll noch bevorzugter Lutein ist.

5. Färbezusammensetzung nach Anspruch 3 oder 4, umfassend 0,01 bis 0,1 Gew.-% des Farbstoffmoleküls mit der Struktur (I), ein pharmazeutisch akzeptables Salz oder ein Hydrat davon, 0,5 bis 3,0 Gew.-% Lutein, 0 bis 0,25 Gew.-% Trypanblau.

6. Färbezusammensetzung nach einem der Ansprüche 3 bis 5 zur Verwendung in einem Verfahren der Augenchirurgie, umfassend das Färben einer Augenmembran oder -struktur, wobei die Augenmembran oder -struktur vorzugsweise die vordere und hintere Kapsel, das Hornhautepithel und - endothel, die epiretinale Membran, die innere Begrenzungsmembran, den Glaskörper oder das hintere Hyaloid umfasst und wobei das Verfahren der Augenchirurgie vorzugsweise die Entfernung einer Augenmembran oder - struktur umfasst.

7. Verfahren zum Färben von Proteinen in einer Probe, umfassend den Schritt des In-Kontakt-Bringens der Probe mit der Färbezusammensetzung nach einem der Ansprüche 3 bis 5, wobei das Färben vorzugsweise durchgeführt wird, um einen oder mehrere analytische Parameter der Proteine zu bestimmen, wobei der analytische Parameter vorzugsweise ausgewählt ist aus der Gruppe bestehend aus Molekulargewicht, Menge und Identität.

8. Verfahren zur Synthese eines Farbstoffmoleküls mit der Struktur (I), wie in einem der Ansprüche 1 oder 2 definiert,
wobei das Verfahren umfasst
a. Bilden einer Reaktionsmischung mit einem pH-Wert von 8 bis 14, umfassend ein protisches Lösungsmittel und ein Molekül der Struktur (III) 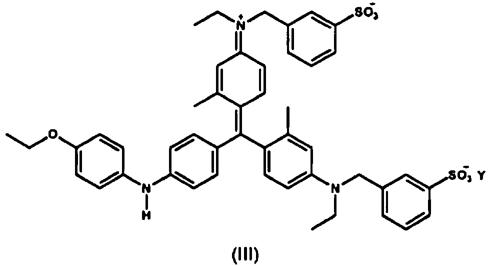 wobei Y ein organisches oder anorganisches Kation darstellt; und
b. Bestrahlen der aus Schritt a. resultierenden Reaktionsmischung mit Mikrowellenstrahlung, umfassend elektromagnetische Wellen mit einer Leistung von 5 W bis 300 W in Gegenwart eines Alkylierungsmittels der Struktur (IV)
R₁-Z (IV)
wobei R₁ eine substituierte oder unsubstituierte Benzylgruppe, wie in Anspruch 1 definiert, darstellt und Z eine Fluchtgruppe darstellt, um das Farbstoffmolekül mit der Struktur (I) zu erhalten, und
wobei das Alkylierungsmittel der Reaktionsmischung in einem oder mehreren Aliquoten im Verlauf von Schritt b. zugesetzt wird.

9. Syntheseverfahren nach Anspruch 8, wobei Y ein organisches oder anorganisches Kation darstellt, ausgewählt aus der Gruppe bestehend aus H⁺, NH₄⁺, einem Ion, ausgewählt aus der Gruppe bestehend aus Na⁺, Mg⁺⁺, K⁺, Ca⁺⁺, einem Lysiniumkation oder einem Argininiumkation.

10. Syntheseverfahren nach einem der Ansprüche 8 oder 9, wobei die Fluchtgruppe Z ausgewählt ist aus der Gruppe der Halogenide.

11. Syntheseverfahren nach einem der Ansprüche 8 bis 10, wobei der Schritt b. bei einer Temperatur von 30°C bis 100°C durchgeführt wird.

12. Syntheseverfahren nach einem der Ansprüche 8 bis 10, ferner umfassend den Schritt der Reinigung des aus Schritt b. resultierenden Farbstoffmoleküls mit der Struktur (I).

## Revendications

1. Molécule de colorant ayant la structure (I) 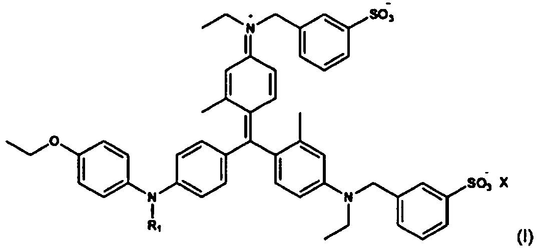 dans laquelle
X représente un cation organique ou inorganique ; et
R₁ représente un groupe de benzyle substitué ou non substitué choisi parmi les groupes de benzyle, 4-méthylbenzyle, 2-méthylbenzyle, 3-méthylbenzyle, 3,5-diméthylbenzyle, 4-chlorobenzyle, 4-isopropylbenzyle, 2-(méthyl)-1,3-diméthylbenzène, 4-(méthyl)benzonitrile, 4-(trifluorométhyl)benzyle, 2,6-dichlorobenzyle, 2-chlorobenzyle, 1-(méthyl)-2,3-diméthylbenzène, 3,4-dichlorobenzyle, 3,5-di-tert-butylbenzyle, 3-(méthyl)benzonitrile, 4-fluorobenzyle, 1-(méthyl)-3-(difluorométhyl)benzène, 3-(trifluorométhyl)benzyle, 4-bromobenzyle, acide 4-(méthyl)benzoïque, 2,3-dichlorobenzyle, 2,5-dichlorobenzyle, 4-iodobenzyle, 3-fluorobenzyle, 3,5-bis(trifluorométhyl)benzyle, 1-(méthyl)-2-(difluorométhyl)benzène, 1-(méthyl)-2,4-diméthylbenzène, 4-(méthyl)-3-méthylbenzonitrile, 4-méthyl-benzamide, 4-(méthylthio)benzyle, alcool 2-(méthyl)benzylique, 2-(trifluorométhyl)benzyle, 2-fluorobenzyle, 3-bromobenzyle, 2-bromobenzyle, 3,5-difluorobenzyle, 4-tert-butylbenzyle, 3-chlorobenzyle, 2-(méthyl)benzonitrile, 1-méthyl-4-(2,2,2-trifluoroéthyl)benzène, bromhydrate de 4-(méthyl)-N,N-diméthylaniline, 2-iodobenzyle, 2,6-difluorobenzyle, 3-iodobenzyle, 3,5-dibromobenzyle, éther méthylique de 3-(méthyl)benzyle, acide 3-(méthyl)benzoïque, 1-(méthyl)-3-chloro-5-fluorobenzène, 3-fluoro-5-méthylbenzyle, 2-(méthyl)-1-méthyl-4-(trifluorométhyl)benzène, 3-fluoro-4-méthylbenzyle, 4-bromo-1-(méthyl)-2-chlorobenzène, 4-fluoro 2-méthylbenzyle, 4-phénoxybenzyle, 4-chloro-3-fluorobenzyle, 1-bromo-2-(méthyl)-3-chlorobenzène, 2-fluoro-4-méthylbenzyle, 4-fluoro-3-méthylbenzyle, acide 2-[4-(méthyl)phényl]propionique, 2-chloro-6-fluorobenzyle, acide 4-(méthyl)phénylboronique, 3-méthoxybenzyle, 2,4,6-trifluorobenzyle, 2,4-difluorobenzyle, 3,4-difluorobenzyle, 4-(méthyl)benzophénone, 2-méthyl-3-(trifluorométhyl)benzyle, 2-chloro-4-fluorobenzyle, 4-(méthyl)benzoate de méthyle, 4-chloro-2-fluorobenzyle, 2-fluoro-3-méthylbenzyle, 3,5-diméthoxybenzyle, 1-bromo-4-(méthyl)-2-chlorobenzène, 5-méthyl-2-(trifluorométhyl)benzyle, 4-chloro-2,6-difluorobenzyle, 4-(méthyl)-chloro-1-fluorobenzène, 5-fluoro-2-méthylbenzyle, 3,4,5-trifluorobenzyle, 2,5-difluorobenzyle, 4-nitrobenzyle, 2-(méthyl)-1-fluoro-3-méthylbenzène, 2-fluoro-5-méthylbenzyle, 3-chloro-5-(trifluorométhyl)benzyle, 2-chloro-5-fluorobenzyle, 4-(difluorométhoxy)benzyle, 2,3-difluorobenzyle, 3-chloro-2-fluorobenzyle, 2-[2-(méthyl)phényl]-2-méthylpropanenitrile, 2-chloro-4-méthoxybenzyle, 4-(méthyl)benzoate d'éthyle, 2,3-difluoro-4-méthylbenzyle, 2,6-difluoro-3-méthylbenzyle, 4-chloro-3-(trifluorométhyl)benzyle, 5-chloro-2-fluorobenzyle, 4-(trifluorométhylthio)benzyle, 2-chloro-5-(trifluorométhyl)benzyle, 4-(méthylsulfonyl)benzyle, 3-(méthyl)benzoate de méthyle, 4-(trifluorométhoxy)benzyle, 3-bromo-5-fluorobenzyle, 4-chloro-2-(trifluorométhyl)benzyle, 3-phénylbenzyle, 2,3,5,6-tétrafluorobenzyle, 6-chloro-2-fluoro-3-méthylbenzyle, 2-méthyl-3-(trifluorométhyl)benzyle, 4-phénoxybenzyle, 2,3,5-trifluorobenzyle, 2,3,6-trifluorobenzyle, 3-nitrobenzyle, 3-(trifluorométhoxy)benzyle, [4-(méthyl)phénoxy]acétonitrile, 2-bromo-1-(méthyl)-4-tert-butylbenzène, 1-bromo-4-(méthyl)-2-fluorobenzène, 2-bromo-4-fluorobenzyle, 2-chloro-6-(trifluorométhyl)benzyle, 1-(méthyl)-2-phénoxybenzène, 4-cyano-2-fluorobenzyle, 3-chloro-2,4-difluorobenzyle, 4-(méthyl)-2-fluorobenzonitrile, 5-bromo-2-(méthyl)-1,3-difluorobenzène, 4-méthyl-2,3,5,6-tétrafluorobenzyle, 2,4-dichloro-5-fluorobenzyle, 2-chloro-6-fluoro-3-méthylbenzyle, 2-chloro-3,6-difluorobenzyle, 5-chloro-2-(trifluorométhyl)benzyle, 3-chloro-2,6-difluorobenzyle, 2,4,5-trifluorobenzyle, 2-phénylbenzyle, 3-fluoro-5-(trifluorométhyl)benzyle, 3-fluoro-4-(trifluorométhyl)benzyle, 3-(difluorométhoxy)benzyle, 4-bromo-2-fluorobenzyle, 2-bromo-4-(méthyl)-1-fluorobenzène, 2,3,4-trifluorobenzyle, 2-bromo-1-(méthyl)-3-fluorobenzène, 1-(méthyl)-3-(méthylsulfonyl)benzène, 4-bromo-3-(méthyl)benzonitrile, 3-méthyl-4-nitrobenzyle, 3-cyano-4-fluorobenzyle, 3-(méthyl)benzoate de tert-butyle, 2-bromo-5-fluorobenzyle, 2-fluoro-4-(trifluorométhyl)benzyle, 2-fluoro-3-(trifluorométhyl)benzyle, 2,3,4,5,6-pentafluorobenzyle, 4-bromo-1-(méthyl)-2-éthylbenzène, 2-bromo-1-(méthyl)-3-(trifluorométhyl)benzène, 2-(méthyl)-4-chloro-1-méthoxybenzène, 3-bromo-2-fluorobenzyle, 1-(méthyl)-2-méthyl-4-(trifluorométhoxy)benzène, 4'-méthyl-2-biphénylcarbonitrile, 4-fluoro-3-(trifluorométhyl)benzyle, 2-fluoro-5-(trifluorométhyl)benzyle, 2-nitrobenzyle, 4-(méthyl)-2-méthoxybenzonitrile, 2-(méthyl)-5-fluorobenzonitrile, 1-(méthyl)-3-méthyl-5-(trifluorométhoxy)benzène, 5-fluoro-2-(trifluorométhyl)benzyle, 1-(méthyl)-2-(difluorométhoxy)benzène, 4-fluoro-2-(trifluorométhyl)benzyle, 4-(méthyl)-3-méthoxybenzonitrile, 4-méthoxy-3-(trifluorométhyl)benzyle, 3-fluoro-4-méthoxybenzyle, 2-(trifluorométhoxy)benzyle, 3-(méthyl)-4-méthoxybenzonitrile, 3-bromo-2-(méthyl)benzonitrile, 4-bromo-1-(méthyl)-2-(trifluorométhyl)benzène, 3-(méthyl)-4-chlorobenzoate de méthyle, 2-(méthyl)-3-fluorobenzonitrile, 1-(méthyl)-3-(4-fluorophénoxy)benzène, 2-(méthyl)-1-fluoro-4-méthoxybenzène, 2-bromo-6-(trifluorométhyl)benzyle, 4-fluoro-3-méthoxybenzyle, 5-fluoro-2-méthoxybenzyle, 3-fluoro-5-méthoxybenzyle, 2-bromo-5-méthoxybenzyle, 2,3,5,6-tétrafluoro-4-(trifluorométhyl)benzyle, 4-(méthyl)phénylacétate de phénacyle, 2-fluoro-6-(trifluorométhyl)benzyle, 2-hydroxy-5-nitrobenzyle, 2-fluoro-méthoxybenzyle, 4-méthyl-N, N-diméthyl-benzènesulfonamide, 4-bromo-2-(méthyl)-1-méthoxybenzène, acétate de tert-butyle [2-(méthyl)phénoxy], 4-(méthyl)-1-éthoxy-2-(trifluorométhyl)benzène, 4-chloro-3-(trifluorométhoxy)benzyle, 2-fluoro-6-méthoxybenzyle, 3-fluoro-4-nitrobenzyle, 2-bromo-1-méthyl-5-méthoxy-3-méthylbenzène, 4-(méthyl)-3-méthoxybenzoate de méthyle, 1-bromo-2-(méthyl)-4,5-diméthoxybenzène, (4-(méthyl)phényl)(4-(prop-2-yn-1-yloxy)phényl)méthanone, (4-(méthyl)phényl)(4-(prop-2-yn-1-yloxy)phényl)méthanone, tert-butyl 2,4-difluoro-3-méthoxybenzyle, 2,6-difluoro-3-méthoxybenzyle, 2-nitro-4-(trifluorométhyl)benzyle, 4-fluoro-3-nitrobenzyle, acide 4-méthyl-3-nitrobenzoïque, 3-bromo-5-(méthyl)benzoate de méthyle, 2-fluoro-5-(trifluorométhoxy)benzyle, 2-bromo-4-(méthyl)benzoate de méthyle, 3-(méthyl)-2-méthoxybenzoate de méthyle, 4-bromo-1-(méthyl)-2-(méthylsulfonyl)benzène, 4-bromo-2-(méthyl)benzoate de méthyle, 4-fluoro-2-nitrobenzyle, 4-bromo-1-(méthyl)-2-nitrobenzène, 2-[(phénylsulfonyl)méthyl]benzyle, 4-bromo-2-(méthyl)benzoate d'éthyle, 2-(méthyl)-4-nitrobenzoate de méthyle, 1-(méthyl)-2-fluoro-3-(trifluorométhoxy)benzène, 3-bromo-2-(méthyl)benzoate de méthyle, 3-éthoxy-2,4-difluorobenzyle, 4,5-diméthoxy-2-nitrobenzyle, 4-[2-(méthyl)phénoxy]tétrahydropyrane, 1-[3-(méthyl)phényl]-1H-pyrrole, 2-[4-(méthyl)phényl]-5-méthyl, acide 4-benzyl-acétique, ledit groupe benzyle substitué ou non substitué étant de préférence choisi parmi le benzyle, le 4-(trifluorométhyl)benzyle, le 3,5-bis(trifluorométhyl)benzyle, l'acide 4-benzylacétique, le 3,4-dichlorobenzyle ou le 4-(tert-butyl)benzyle.

2. Molécule de colorant selon la revendication 1, dans laquelle X représente un cation organique ou inorganique choisi dans le groupe constitué par H⁺, NH₄⁺, un ion choisi dans le groupe constitué par Na⁺, Mg⁺⁺, K⁺, Ca⁺⁺, un cation lysinium ou un cation argininium.

3. Composition de coloration comprenant la molécule de colorant ayant la structure (I) selon la revendication 1 ou 2, un sel acceptable du point de vue pharmaceutique, ou un hydrate de celle-ci, la composition comprenant de préférence de 0,0001 % à 0,5 % en poids de ladite molécule de colorant ayant la structure (I), un sel acceptable du point de vue pharmaceutique, ou un hydrate de celle-ci.

4. Composition de coloration selon la revendication 3, comprenant en outre au moins un agent d'ajustement de la densité, dans laquelle ledit agent d'ajustement de la densité est de préférence choisi dans le groupe constitué de la D₂O, des monosaccharides, des disaccharides, des polysaccharides, des xanthophylles et des polymères, dans laquelle ledit agent d'ajustement de la densité comprend plus préférablement au moins une xanthophylle et dans laquelle ladite xanthophylle est encore plus préférablement de la lutéine.

5. Composition de coloration selon la revendication 3 ou 4, comprenant de 0,01 % à 0,1 % en poids de la molécule de colorant ayant la structure (I), un sel acceptable du point de vue pharmaceutique ou un hydrate de celui-ci, de 0,5 % à 3,0 % en poids de lutéine, de 0 % à 0,25 % en poids de bleu de trypan.

6. Composition de coloration selon l'une quelconque des revendications 3 à 5, destinée à être utilisée dans une méthode de chirurgie ophtalmique comprenant la coloration d'une membrane ou d'une structure oculaire, dans laquelle la membrane ou la structure oculaire comprend de préférence la capsule antérieure et postérieure, l'épithélium et l'endothélium cornéens, la membrane épirétinienne, la membrane limitante interne, le vitré ou la hyaloïde postérieure et dans laquelle ladite méthode de chirurgie ophtalmique comprend de préférence le retrait d'une membrane ou d'une structure oculaire.

7. Procédé de coloration des protéines dans un échantillon comprenant l'étape qui consiste à mettre au contact dudit échantillon la composition de coloration selon l'une quelconque des revendications 3 à 5, dans lequel ladite coloration est de préférence effectuée pour déterminer un ou plusieurs paramètres analytiques des dites protéines, lesdits paramètres analytiques étant de préférence choisis dans le groupe constitué par la masse moléculaire, la quantité, l'identité.

8. Procédé de synthèse d'une molécule de colorant ayant la structure (I) tel que défini dans l'une quelconque des revendications 1 ou 2,
le procédé comprenant :
a. former un mélange réactionnel ayant un pH de 8 à 14 comprenant un solvant protique et une molécule ayant la structure (III) ; 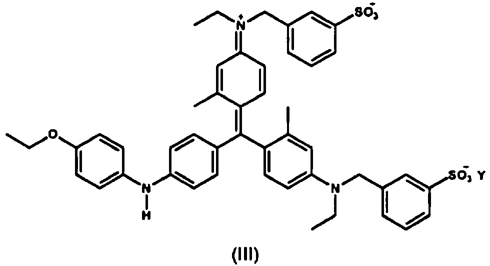 dans lequel Y représente un cation organique ou inorganique ; et
b. irradier le mélange réactionnel résultant de l'étape a. avec un rayonnement à micro-ondes comprenant des ondes électromagnétiques ayant une puissance de 5 W à 300 W en présence d'un agent d'alkylation ayant la structure (IV) ;
R₁-Z (IV)
dans lequel R₁ représente un groupe de benzyle substitué ou non substitué tel que défini dans la revendication 1 et Z représente un groupe partant ; de façon à obtenir ladite molécule de colorant ayant la structure (I) ; et
dans lequel l'agent d'alkylation est ajouté au mélange réactionnel en une ou plusieurs aliquotes au cours de l'étape b.

9. Procédé de synthèse selon la revendication 8, dans lequel Y représente un cation organique ou inorganique choisi dans le groupe constitué par H⁺, NH₄⁺, un ion choisi dans le groupe constitué par Na⁺, Mg⁺⁺, K⁺, Ca⁺⁺, un cation lysinium ou un cation argininium.

10. Procédé de synthèse selon l'une quelconque des revendications 8 ou 9, dans lequel ledit groupe partant Z est choisi dans le groupe constitué par les halogénures.

11. Procédé de synthèse selon l'une quelconque des revendications 8 à 10, dans lequel ladite étape b. est réalisée à une température comprise entre 30 °C et 100 °C.

12. Procédé de synthèse selon l'une quelconque des revendications 8 à 10, comprenant en outre l'étape consistant à purifier ladite molécule de colorant de structure (I) issue de l'étape b.
